(19) Europäisches Patentamt
European Patent Office
Office européen des brevets

(11) Publication number: **0 099 122**
**B1**

(12) **EUROPEAN PATENT SPECIFICATION**

(45) Date of publication of patent specification: **22.10.86**

(21) Application number: **83106847.3**

(22) Date of filing: **12.07.83**

(51) Int. Cl.[4]: **A 61 K 37/02,** A 61 K 31/13, A 61 K 31/075, A 61 K 31/10, A 61 K 31/34, A 61 K 31/38, A 61 K 31/44

(54) **Compositions comprising pepstatin and an histamine H2-receptor antagonist having an enhanced antiulcer activity.**

(30) Priority: **12.07.82 US 397277**

(43) Date of publication of application:
**25.01.84 Bulletin 84/04**

(45) Publication of the grant of the patent:
**22.10.86 Bulletin 86/43**

(84) Designated Contracting States:
**AT BE CH DE FR GB IT LI LU NL SE**

(56) References cited:
**EP-A-0 030 092**
**EP-A-0 056 973**
**US-A-3 840 516**
**US-A-4 062 863**
**US-A-4 091 093**
**US-A-4 101 650**

(73) Proprietor: **Bristol-Myers Company**
**345 Park Avenue**
**New York New York 10154 (US)**

(72) Inventor: **Buyniski, Joseph P.**
**302 Rugby Road**
**Syracuse New York (US)**
Inventor: **Cavanagh, Robert L.**
**7684 Stonehedge Lane**
**Manlius New York (US)**
Inventor: **Gordon, Maxwell**
**513 Standish Drive**
**Syracuse New York (US)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

**EP 0 099 122 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

In the treatment of peptic ulcers in warm-blooded animals, the concomitant administration of the pepsin-complexing agent, pepstatin, and a histamin $H_2$-receptor antagonist of the formula I:

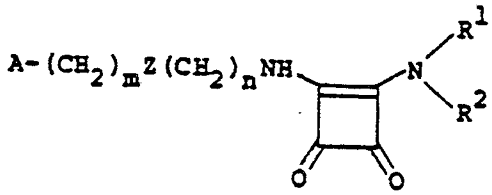

I

wherein A, m, Z, n, $R^1$ and $R^2$ are as defined below, provides enhanced anticuler activity, reduces the amount of the compound of Formula I necessary for effective treatment and thereby reduces the side effect liability of the compound of Formula I. This invention relates to pharmaceutical compositions containing pepstatin and at least one compound of Formula I for treating peptic ulcers in warm-blooded animals.

The precise cause of peptic ulceration in man is unknown although gastric acid is considered to be one of the essential factors in the etiology of this disease. It recently was discovered that gastric acid secretion is mediated, at least in part, by histamin $H_2$-receptors located on parietal cells in the gastric mucosa and the gastric acid output induced by all secretagogues could be antagonized by specific antagonists of these receptors [Black, J. W. *et al.*, Nature, *236*, 385—390 (1972); Brimblecombe, R. W. *et al.*, J. Int. Med. Res., *3*, 86—92 (1975)]. The first successful comercial histamine $H_2$-receptor antagonist, cimetidine (II)

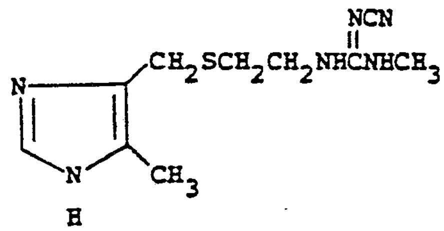

II

is now in widespread use as an antiulcer agent. A more recently introduced histamine $H_2$-receptor antagonist, ranitidine (III)

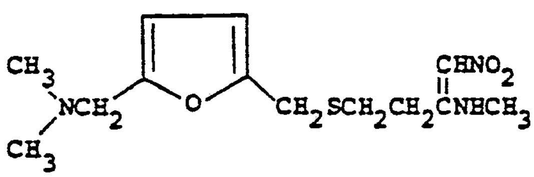

III

is now being sold and used in several countries of the world.

The role of the proteolytic enzyme, pepsin, in the etiology of ulceration is not completely understood. Pepsin has been shown to play a major role in the development of experimentally inducted ulcers in animals, but this may be due to lesion enlargement by means of pepsin digestion, of necrotic tissue rather than by causing the initial damage. It is also possible that pepsin is entirely responsible for the erosions and that the acid produces pain and retards healing.

1) Umezawa, H. *et al.*, in J. Antibiotics, *23*, 259—262 (1970), disclose the pentapeptide, pepstatin, which has the structure

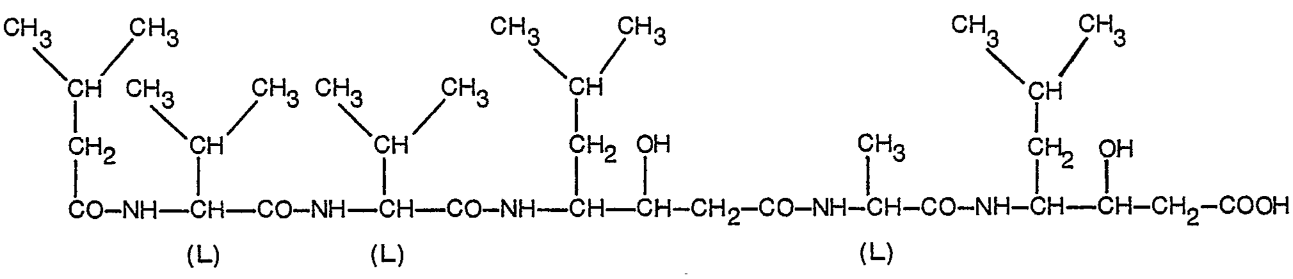

and which is a specific complexing agent for the enzyme pepsin. Pepstatin was found to prevent the formation of stomach ulcers in the pylorus ligated (Shay) rat.

2) Miyawaki, *et al.*, in Nagano-ken Noygo Sogo Shikenjo Chikusan Shikenjo Kenkyu Hokoku, *14*, 14—25 (1977) [as reported in Chemical Abstracts, *90*, 34373X (1979)] reported that the addition of pepstatin to the

feed at >50 ppm inhibited pepsin activity in the gastric juice of swine and prevented the occurrence of ulcers.

3) Bonnevie, O. *et al.*, in Gut, *20*, 624—628 (1979), report the results of a double-blind randomized clinical trial of pepstatin versus placebo in patients having duodenal ulcers. Pepstatin was administered in 100 mg doses, given seven times a day, this dosage being sufficient to inhibit the peptic activity of gastric juice for 18 hours a day. They found no significant difference between pepstatin and placebo in the healing or symptomatology of duodenal ulcer.

4) Svendsen, L. R. *et al.*, in Scand. J. Gâstroent., *14*, 929—932 (1979), report the results of a double-blind randomized clinical trial of pepstatin versus placedo in patients having gastric ulcer. Pepstatin was administered in 100 mg doses seven times a day. They were not able to detect any influence of pepstatin either on the healing or on the symptomatology of gastric ulcer.

5) Strauss, R. J. *et al.*, in Surg. Forum, *28*, 361—363 (197), disclose that, in stress ulceration tests in rats, two or more days of pretreatment with either cimetidine or carbenoxolone significantly decreased ulcer formation. When the two agents were given together, significant ulcer reduction was found after only one-half day of predosing. Carbenoxolone is not an antisecretory or anti-pepsin agent, but acts by stimulating gastric mucus synthesis.

6) Dajani, E. Z. *et al.*, in J. Pharmacol. Exp. Ther., *210*, 373—377 (1979), disclose that, in stress ulceration tests in rats, a combination of cimetidine and propantheline bromide produced synergistic antiulcer activity and that a combination of cimetidine and thiopropazate hydrochloride produced additive antiulcer activity. Propantheline bromide (an anticholinergic agent) and thiopropazate hydrochloride (a tranquilizer) each act by inhibiting gastric secretion.

7) British Medical Journal, 95—96 (1980) reviews the results obtained in a large number of studies with various antiulcer agents. With regard to pepsin antagonists, it states:

> "The results of using pepsin antagonists have been uniformly disappointing. Amylopectin showed no significant benefit in patients with duodenal ulcer, and sucralfate showed none in those with gastric ulcer. Even pepstatin, the most potent in-vitro and in-vivo pepsin antagonist, was ineffective in a formal controlled trial in healing duodenal ulcer and in preventing recurrent bleeding in patients admitted with haematemesis and melaena."

8) U.S. 4,101,650 discloses long-acting pepstatin floating minicapsules comprising center particles of sodium bicarbonate coated with a water-soluble film-coating agent, which are further coated with pepstatin and a water-soluble film coating agent. Because of the release of carbon dioxide in gastric juice, these minicapsules float in the stomach and provide pepsin suppression from 3—5 hours, as compared with about 1 hour for plain pepstatin.

9) U. S. Patent 4,062,863 discloses histamine $H_2$-receptor antagonists of the formula

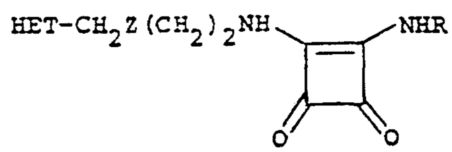

wherein R is hydrogen, (lower)alkyl or $(CH_2)_2Z'CH_2$—HET'; z and Z' each are sulfur or methylene; and HET and HET' each are an imidazole ring optionally substituted by methyl or bromo, a pyridine ring optionally substituted by hydroxy, methoxy, chloro or bromo, a thiazole ring or an isothiazole ring, and pharmaceutically acceptable acid addition salts thereof. U.S. Patents 4,120,968, 4,120,973 and 4,166,857 are divisionals thereof which have substantially the same disclosure.

10) Published European Patent Application No. 30,092 discloses histamine $H_2$-antagonists of the formula

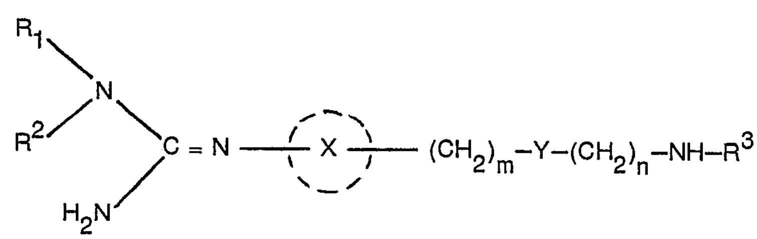

in which

$R^1$ and $R^2$ are hydrogen or optionally halogen-substituted alkyl, cycloalkyl or cycloalkylalkyl, provided that at least one of $R^1$ and $R^2$ is halogen-substituted alkyl, cycloalkyl or cycloalkylalkyl;

X is a phenyl ring with 1 or 2 optional substitutents or a 5- or 6-membered heterocyclic aromatic ring containing 1, 2 or 3 heteroatoms selected from O, N and S, the heterocyclic ring, where possible, having 1 optional substituent, which optional substituents are halogen, alkyl, alkoxy, alkylthio, trifluoromethyl, hydroxy and amino;

Y is O, S, a direct bond, methylene, *cis* or *trans* vinylene, sulfinyl or $NR^4$ in which $R^4$ is H or alkyl;
m is 0 to 4 and n is 1 to 5;

$R^3$ is *inter alia* AB in which A is *inter alia* a 3,4-dioxocyclobuten-1,2,-diyl radical and B is *inter alia* the radical $NR^7R^8$ in which $R^7$ and $R^8$ are *inter alia* hydrogen, alkyl, haloalkyl, alkoxycarbonyl, alkenyl, alkynyl, (primary hydroxy)alkyl or (primary amino)alkyl, or $R^8$ and $R^9$, together with the nitrogen atom to which they are attached, may be a 5- or 6- membered ring which optionally contains O or $NR^9$ in which $R^9$ is H or alkyl.

The patents listed under items 9) and 10) above are cited as illustrative of those disclosing histamine $H_2$-receptor antagonists containing a 1,2-di(optionally substituted)amino-cyclobutene-3,4-dione nucleus. Other such patents exist, but none discloses that their compounds provide enhanced antiulcer activity when administered with pepstatin, and none discloses the compounds of Formula I utilized herein.

This invention relates to a pharmaceutical composition comprising a mixture of the pepsin-inhibiting agent, pepstatin, and at least one histamine $H_2$-receptor antagonist of the formula

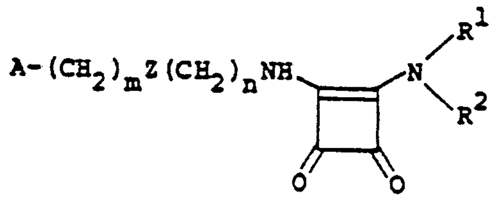

I

wherein $R^1$ and $R^2$ are each independently hydrogen or $C_1$—$C_6$-alkyl, and, when $R^1$ is hydrogen, $R^2$ also may be allyl, propargyl, $C_3$—$C_7$-cycloalkyl-$C_1$—$C_6$-alkyl, Cyclo-$C_3$—$C_7$-alkyl, cyano -$C_1$—$C_6$-alkyl, 2-fluoroethyl, 2,2,2-trifluoroethyl, hydroxy, 2,3-dihydroxypropyl,

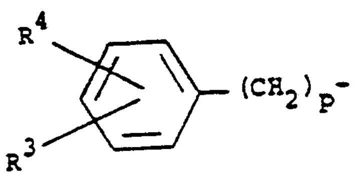

or

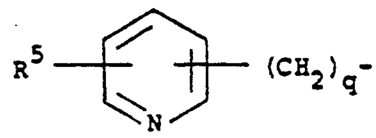

in which p is an integer of from 1 to 6 inclusive, 1 is an integer of from 1 to 6 inclusive, $R^3$ and $R^4$ each are independently hydrogen, $C_1$—$C_6$-alkyl, hydroxy, $C_1$—$C_6$-alkoxy or halogen, and, when $R^3$ is hydrogen, $R^4$ also may be trifluoromethyl, or $R^3$ and $R^4$, taken together may be methylenedioxy, $R^5$ is hydrogen, $C_1$—$C_6$-alkyl, $C_1$—$C_6$-alkoxy, hydroxy, amino or halogen;

m is an integer of from 0 to 2 inclusive;
n is an integer of from 2 to 5 inclusive;
Z is sulfur, oxygen or methylene; and
A is

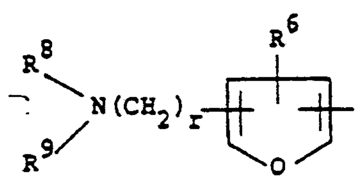

,

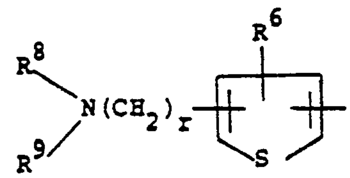

,

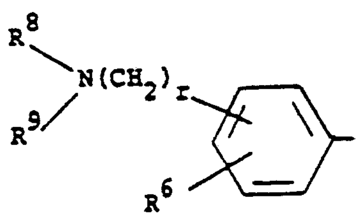

or

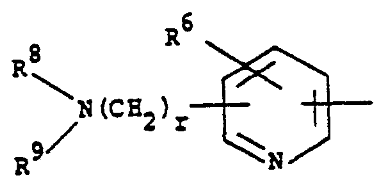

in which $R^6$ is hydrogen, $C_1$—$C_6$-alkyl, $C_1$—$C_6$-alkoxy or halogen;

r is an integer of from 1 to 4 inclusive; and

$R^8$ and $R^9$ each are independently hydrogen, $C_1$—$C_6$-alkyl, allyl, propargyl, $C_1$—$C_6$-alkoxy-$C_1$—$C_6$-alkyl in which the $C_1$—$C_6$-alkoxy moiety is at least two carbon atoms removed from the nitrogen atom, $C_3$—$C_7$-cycloalkyl, or phenyl-$C_1$—$C_6$-alkyl, provided that $R^8$ and $R^9$ may not both be $C_3$—$C_7$-cycloalkyl, or $R^8$ and $R^9$ taken together with the nitrogen atom to which they are attached, may be pyrrolidin-l-yl, methylpyrrolidin-l-yl, dimethylpyrrolidin-l-yl, morpholino, thiomorpholino, piperidino, methylpiperidino, dimethylpiperidino, hydroxypiperidino, 4-methyl-l-piperazinyl, 1,2,3,6-tetrahydropyrid-l-yl, 3-pyrrolin-l-yl, homo-

piperidino, heptamethyleneimino, octamethyleneimino or 3-azabicyclo[3,2,2]-nonane, or a nontoxic, pharmaceutically acceptable acid addition salt, hydrate or solvate thereof.

With the compositions of the invention it is possible to treat peptic ulcers in a warm-blooded animal in need of such treatment by concomitantly administering to said animal a peptic activity-inhibiting amount of pepstatin and an effective antiulcerogenic amount of at least one compound of Formula I, or a pharmaceutically acceptable acid addition salt, hydrate or solvate thereof.

Thus it is possible to reduce the amount of the compound of Formula I necessary for effective treatment of peptic ulcers in a warm-blooded animal by concomitantly administering to said animal a peptic activity-inhibiting amount of pepstatin and an effective antiulcerogenic amount of at least one compound of Formula I, or a pharmaceutically acceptable acid addition salt, hydrate or solvate thereof.

The compounds of Formula I are not themselves our invention, but are the invention of our colleagues, Aldo A. Algieri and Ronnie R. Crenshaw.

The compounds of Formula I are relatively nontoxic substances, as demonstrated by pharmacological studies in animals of some of the compounds. These studies showed a toxicity profile substantially the same as that of the commercial $H_2$-antagonist cimetidine. Although widespread human usage of cimetidine has demonstrated it to be a relatively safe drug with a low incidence of side-effects, it would, of course, be desirable to even further reduce the side-effect liability of such a drug. The compounds of Formula I have been shown by various animal studies to be from about equipotent to about 150 times as potent as cimetidine as inhibitors of gastric acid secretion, depending on the animal model and route of administration, with the preferred compounds of Examples 1 and 9 being about 20—25 times more potent as cimetidine by the oral route. Based on this potency difference, the oral dosage of the compounds of Eamples 1 and 9 would be about 1/20 to 1/25 that of cimetidine, thus reducing the expected incidence of side effects. The usual oral dosage of cimetidine is 300 mg, given four times a day, while the usual dosage of the compounds of Examples 1 and 9 is about 12—15 mg, given four times a day. It was an object of this invention to further reduce the necessary dosage of the compounds of Formula I by the concomitant administration of a peptic activity-inhibitory amount of pepstatin. As will be shown below, such concomitant administration provides about a three-fold increase in potency compared with the administration of an equal amount of the compound of Formula I alone, thus permitting a further three-fold reduction in the dosage of the compound of Formula I.

Pepstatin has also been shown by pharmacological studies in animals to be a relatively nontoxic substance; its $LD_{50}$ exceeded 3000 mg/kg in all animal species studied [Svendsen, L. B. *et al.*, Scand. J. Gastroent., *11*, 459—463 (1976)]. It is essentially unabsorbed upon oral administratioon; no side effects were observed in human patients with ulcer dyspepsia receiving daily oral doses of 700 mg of pepstatin for up to three months [Svendsen, L. B. *et al.* (1976) supra]. Pepstatin does not inhibit the production of pepsin but inhibits peptic activity by forming a 1:1 pepsin-pepstatin complex which is devoid of proteolytic activity.

In patients with ulcer dyspepsia, it has been demonstrated that pepstatin inhibits gastric peptic activity, but has no effect on the gastric acidity [Svendsen, L. B. *et al.*, Scan. J. Gastroent. *11*, 459—463 (1976)]. In contrast, the histamine $H_2$-receptor antagonist cimetidine has been shown to antagonize both basal and stimulated gastric acid secretion in normal volunteers [Burland, W. L. *et al.*, Brit. J. Clin. Pharmacol., *2*, 481—486 (1975)] and in patients with duodenal ulcer [Longstreth, G. F. *et al.*, New England J. Med., *294*, 801—804 (1976)], but its effect on pepsin secretion is less marked [Binder, H. J. and Donaldson Jr., R. M., Gastroenterology, *74*, 371—375 (1978)]. The results of those studies indicate that pepstatin and cimetidine act by different mechanisms. The inhibitory effect of the compounds of Formula I on gastric acid secretion is also significantly greater than on pepsin activity, and in this respect its pharmacological profile is similar to that of cimetidine.

In the tests described below, gastric erosions produced in rats by the oral instillation of 1.0 ml of 0.75N HCl were compared with those in rats which had been pretreated with the most preferred compound of Formula I (of Example 1) or with that compound and pepstatin. Comparison tests utilizing pretreatment with the newer commercial product, ranitidine, and with ranitidine and pepstatin, are also shown. Ranitidine is about 4 and 6 times as potent as cimetidine as an inhibitor of gastric acid secretion by the oral route in the rat and dog, respectively.

Experimental Methods

A modification of the method of Robert *et al.*, [Gastroenterology, *77*, 433—443 (1979)] was employed to produce gastric erosions. Adult male, Long Evans rats weighing 280—300 g (Blue Spruce Farms, Alton, New York) were used. The animals were individually caged and food and water was removed 24 and 18 hours, respectively, prior to testing. On the following day, the test compound was administered orally to the animals 30 minutes before 1.0 ml of 0.75N HCl was instilled into the stomach by gavage. Animals treated with the combination of the test compound and pepstatin received the test compound 30 minutes before, and a fixed amount of pepstatin (20 mg/kg p.o.) 10 minutes before, the hydrochloric acid was administered. Previous studies in our laboratories had shown that this dose of pepstatin (20 mg/kg p.o.) completely inhibited pepsin activity and antagonized ulcer formation in the 18 hour pylorus ligated rat. One hour after receiving the HCl solution, the animals were sacrificed with an intraperitoneal injection of 0.2 ml of T-61®, a euthanasia solution (National Laboratories Corp.).

The stomachs were removed from the animals, cut along the greater curvature, opened, rinsed with saline and pinned out flat in a standard position for macroscopic examination and scoring of erosions. The stomachs were photographed with a Polaroid® Close Up camera (Polaroid Corporation) and scoring was determined from the photographs. For scoring purposes, only those erosions with a minimum length of 1 mm were considered. The severity of gastric ulceration was defined for each animal as the sum of the maximum continuous lengths (in mm) of the erosions satisfying the above criteria. Percent inhibition of lesion formation was defined as

$$\frac{(\text{mm erosion, vehicle control}) - (\text{mm erosion, test agent})}{(\text{mm erosion, vehicle control})} \times 100.$$

Data were anaylzed using the t-test for unpaired data and $ED_{50}$ values were calculated from the dose response data using probit analysis [Finney, Probit Analysis, 3rd ed., University Press, Cambridge, England (1971)].

The compound of Example 1 and ranitidine (synthesized by the Medicinal Chemistry Research Department of Bristol Laboratories Division of Bristol-Myers Company) were dissolved in one equivalent of HCl and the pH adjusted to 5.5 with NaOH. A suspension of pepstatin (Banyu Pharmaceutical Co. Ltd.) in water was made by homogenizing the compound with a few drops of Tween-80® (Atlas Chemical Industries). Each of these compounds were administered orally by gavage in a volume of 2 ml/kg.

Test Results

The instillation of HCl to untreated rats caused extensive gastric erosions consisting of elongated bands 1—10 mm long by 1—3 mm wide. These erosions were located primarily in the corpus (portion of the stomach which secretes acid and pepsin), while the antrum was not as severely affected and no lesions were observed in the forestomach (the non-secretory portion). These findings are similar to those reported by Robert *et al.*, in their initial description of this procedure.

Pretreatment of the rats with 25, 50 or 75 mg/kg dose of the compound of Example 1 prior to instillation of the HCl decreased the formation of gastric erosions in a dose-related manner. Pretreatment of the rats with the compound of Example 1 plus 20 mg/kg of pepstatin significantly enhanced the inhibitory effect of the compound of Example 1 at doses of 50 and 75 mg/kg. An enhancement was also seen at 25 mg/kg, but the difference was not statistically significant ($.05 < p < .10$). Figure 1 shows, in graphic form, the percent of reduction in gastric erosions over that of the control rats (no pretreatment), which was obtained at each of the dosage levels of the compound of Example 1 alone and the compound of Example 1 plus pepstatin. When the data shown in Figure 1 were analyzed by probit analysis according to Finney, it was shown that the response to the compound of Example 1 was linear with respect to the log dose, and that the addition of pepstatin shifted the dose response to the left in a parallel manner. These data are shown in Figure 2. It may be seen from Figure 2 that the $ED_{50}$ values from the compound of Example 1 alone and the compound of Example 1 plus pepstatin combination were found to be 115 and 33 mg/kg, respectively, thus showing that the combination had more than three times the potency of the compound of Example 1 alone.

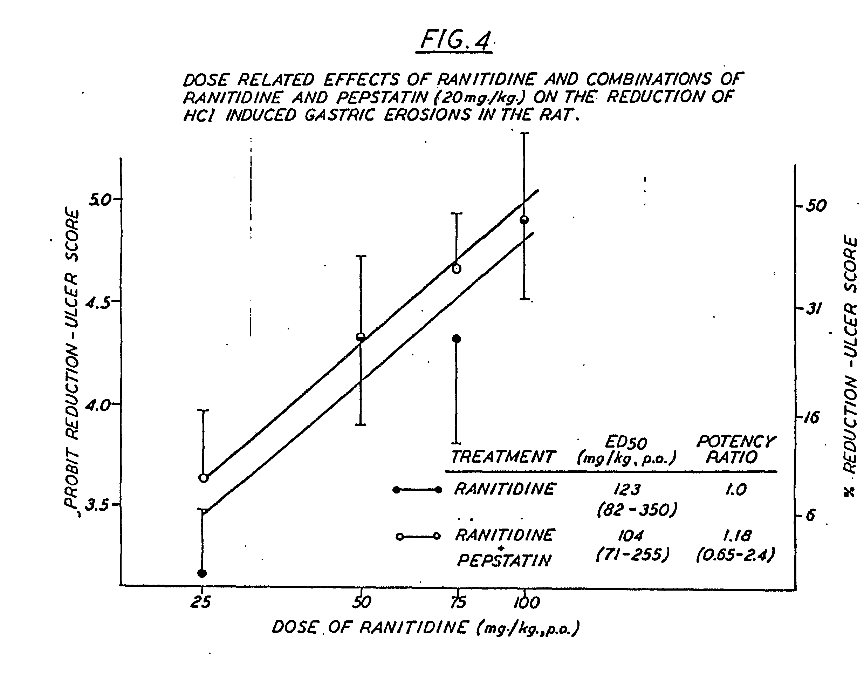

Pretreatment of rats with a 25, 50, 75 or 100 mg/kg dose of ranitidine prior to instillation of the HCl similarly decreased the formation of gastric erosions in a dose-related manner. No enhancement of the inhibitory effect over that of ranitidine alone occurred when pepstatin was given along with ranitidine. Figure 3 shows, in graphic form, the percent reduction in gastric erosions over that of the control rats (no pretreatment), which was obtained at each of the dosage levels of ranitidine alone and ranitidine plus pepstatin. When the data shown in Figure 3 were analyzed by probit analysis according to Finney, it was shown that the response to ranitidine was linear with respect to the log dose of the compound and that the addition of pepstatin shifted the dose response to the left in a parallel manner. These data are shown in Figure 4. It may be seen from Figure 4 that the $ED_{50}$ values for ranitidine alone and the ranitidine-pepstatin combination were found to be 123 and 104 mg/kg, respectively. The ratio of potencies of the combination to ranitidine alone was 1.18, which was not statistically different from unity.

The data from these tests are summarized in Table 1. It may be seen that the $ED_{50}$'s for the compound of Example 1 and ranitidine, given alone, were substantially the same. However, when each of these compounds were administered with pepstatin, the compound of Example 1 showed an enhancement of protection against ulcer formation (by a factor of 3.5) while ranitidine showed no significant ehancement of protection. Thus, the potency of the compound of Example 1 plus pepstatin was approximately three times that of ranitidine plus pepstatin.

TABLE 1
Comparison of the Compound of Example 1 and Rantitidine in Inhibiting HCl-Induced Formation of Gastric Lesions, Alone or in Combination with Pepstatin

| | $ED_{50}$ (mg/kg) for Inhibition of Ulcer Formation | | |
|---|---|---|---|
| Compound | Alone | with Pepstatin | Potency Radio[a] |
| Compound of Example 1 | 115 | 33 | 3.5 |
| Ranitidine | 123 | 104 | 1.18 |

[a]Ratio of potency (cpd. alone) to potency of combination (cpd. plus pepstatin).

Antagonism of $H_2$-receptors and the subsequent anti-secretory effect probably is not the mechanism of the antiulcer effect in this test, since exogenous HCl is being supplied. Pepstatin is only poorly absorbed following oral administration as animal studies have shown that more than 90 percent of the compound is excreted in the feces within 72 hours. Therefore, the inhibition of proteolytic activity following oral administration of pepstatin is due primarily to a local effect of this compound.

In order to obtain the maximum benefit of the present invention, it is desirable that the dosage of pepstatin be such that there is substantially complete inhibition of gastric pepsin activity for as long a period of the day as practical. When pepstatin was administered to ulcer patients in 100 mg doses seven times a day (with meals, two hours after meals and at bedtime), pepsin activity was inhibited for 18 hours a day.

In one preferred embodiment of this invention, pepstatin is administered in dosages of about 100 mg seven times a day. In another preferred embodiment of this invention, pepstatin is administered in dosages of about 175 mg four times a day. In a more preferred embodiment of this invention the pepstatin is administered in the form of floating minicapsules as described in U.S. Patent 4,101,650. The pepstatin floating minicapsules provide pepsin suppression for about 3—5 times as long as plain pepstatin and, in this form, may be administered, for example, four times a day in a dosage of floating minicapsules containing about 100 mg of pepstatin.

The dosage of the compounds of Formula I to be administered concomitantly with pepstatin will depend not only on such factors as the weight of the patient, but also on the degree of gastric acid inhibition desired and the potency of the particular compound being utilized. The decision as to the particular dosage to be employed is within the discretion of the physician. In the Heidenhain Pouch Dog test described below, cimetidine has an oral $ED_{50}$ of approximately 3.3 µmoles/kg. The usual human adult oral dose of cimetidine is 300 mg, given four times a day. The usual human adult starting oral dosages of the compounds of Formula I (without pepstatin) are readily determined from their oral $ED_{50}$ in this same test. Thus, if the oral $ED_{50}$ of a particular compound of Formula I is 0.33 µmoles/kg, the usual starting dosage (without pepstatin) would be approximately 30 mg, given four times a day, etc. When administered concomitantly with pepstatin, the usual starting dose would be approximately 15 mg, given four times a day. Similar calculations may be made for parenteral dosages. These starting dosages (and the number of times administered per day) may, of course, be varied by titration of the dosage to the particular circumstances of the specific patient.

It will be appreciated by those skilled in the art that, to obtain the benefits of the present invention, it is not necessary to physically combine the compound of Formula I and pepstatin in a single unitary dosage form. Not only may the two active ingredients be taken separately, but they may even be given by different routes of administration. Although pepstatin provides its effects by local action in the stomach and must be given orally, the compound of Formula I may be given orally or parenterally. For convenience, however, it usually is preferred to administer it orally.

The treatment of peptic ulcers in a warm-blooded animal in need of such treatment involving the compositions according to the invention comprises concomitantly administering to said animal a peptic activity-inhibiting amount of pepstatin and an effective antiulcerogenic amount of at least one compound of Formula I, or a pharmaceutically acceptable acid addition salt, hydrate or solvate thereof. In man, the usual dosage of the preferred compounds of Formula I is from about 2 to about 100 mg (and most preferably from about 4 to about 50 mg), given three or four times (and most preferably four times) a day. With the most preferred compound of Formula I (the compound of Example 1), the usual dosage is from about 2 to about 15 mg, and preferably from about 4 to about 10 mg. The preferred dosage of pepstatin in man is from about 100 mg when administered about seven times a day, to about 175 mg when administered about four times a day. However, in a more preferred embodiment of this invention, when the pepstatin is in the form of pepstatin floating minicapsules, the preferred dosage is that amount of minicapsules containing about 100 mg of pepstatin, administered about four times a day.

There is provided by the present invention a pharmaceutical composition useful in the treatment of peptic ulcers which comprises a peptic activity-inhibiting amount of pepstatin and an effective antiulcerogenic amount of at least one compound of Formula I, or a pharmaceutically acceptable acid addition salt, hydrate or solvate thereof, and a pharmaceutically acceptable carrier. In a preferred embodiment the composition contains from 2 to 100 mg (and most preferably from 4 to 50 mg) of one of the preferred compounds of Formula I and from 100 to 175 mg of pepstatin. In the most preferred embodiment, the composition contains from 2 to 15 (and most preferably from 4 to 10) mg of the compound of Example 1, plus from 100 to 175 mg of pepstatin. Preferably the compositions according to the invention are in unitary dosage form.

As used herein, reference to a pharmaceutically acceptable acid addition salt of a compound of Formula I means the mono or di-salt with a nontoxic pharmaceutically acceptable organic or inorganic acid. Such acids are well known and include hydrochloric, hydrobromic, sulfuric, sulfamic, phosphoric, nitric, maleic, fumaric, succinic, oxalic, benzoic, methanesulfonic, ethanedisulfonic, benzenesulfonic, acetic, propionic, tartaric, citric, camphorsulfonic and levulinic. The salts are made by methods known in the art.

In practicing the present invention, a wide variety of pharmaceutical forms may be employed for the administration of the compound of Formula I and pepstatin, or the pharmaceutical composition containing both entities. Thus, if a solid carrier is used, the preparations may be tableted, placed in a hard gelatin capsule in powder or pellet form, or in the form of a troche or lozenge. If a liquid carrier is used, the preparations may be in the form of a soft gelatin capsule, syrup, emulsion, aqueous or non-aqueous suspension or, in the case of the compounds of Formula I, a sterile solution or suspension for injection. These pharmaceutical dosage forms are prepared by conventional techniques.

Preparation of Compounds of Formula I

The compounds of Formula I may be prepared by various alternative procedures, utilizing as a starting material a compound of the Formula

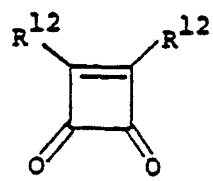

IV

in which $R^{12}$ is a good leaving group such as halogen, phenoxy, substituted phenoxy, alkoxy or the like. Suitable leaving groups are well-known to those skilled in the art. Preferably, $R^{12}$ is $C_1$—$C_6$-alkoxy, and especially methoxy and ethoxy.

The compounds of Formula I may be prepared from a compound of Formula IV by various alternative reaction schemes, as shown below in Reaction Schemes 1 and 2.

Reaction Scheme 1

Compound IV

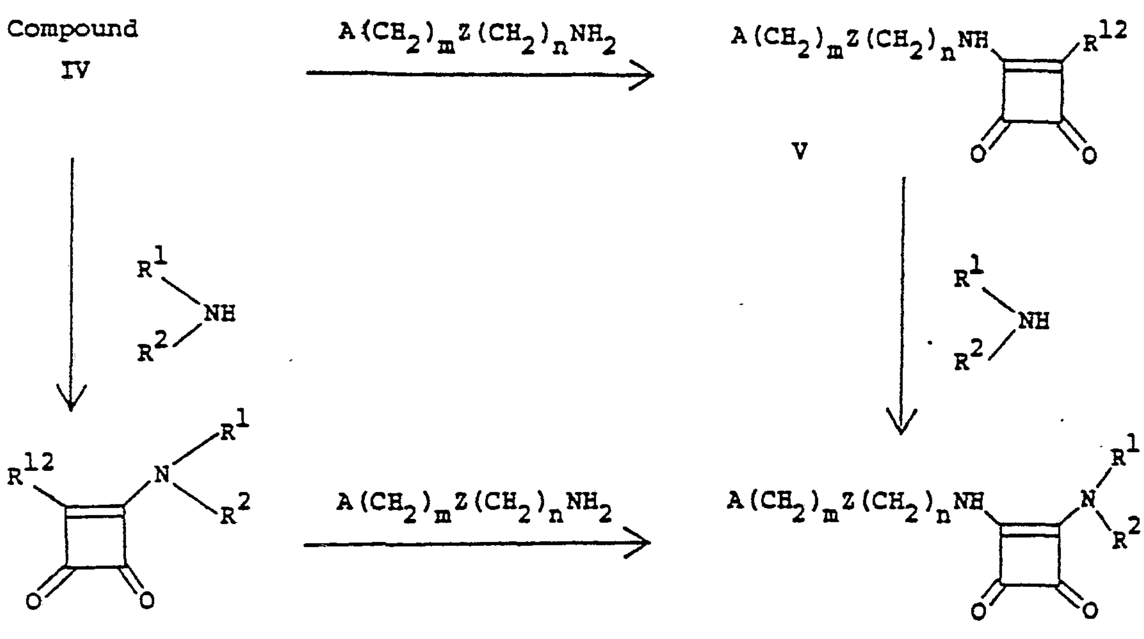


V

VI

I

The reactions are conducted in an inert organic solvent; methanol is a convenient and readily available solvent. The reaction temperature is not critical. Most starting materials are quite reactive and the reaction preferably is conducted at a temperature below room temperature, e.g. 0—10°C. With some less reactive compounds it is convenient to conduct the reaction at room temperature. Sometimes it is desirable to subsequently raise the temperature of the reaction mixture (e.g. to 50—60°C) to complete the reaction.

Reaction Scheme 2

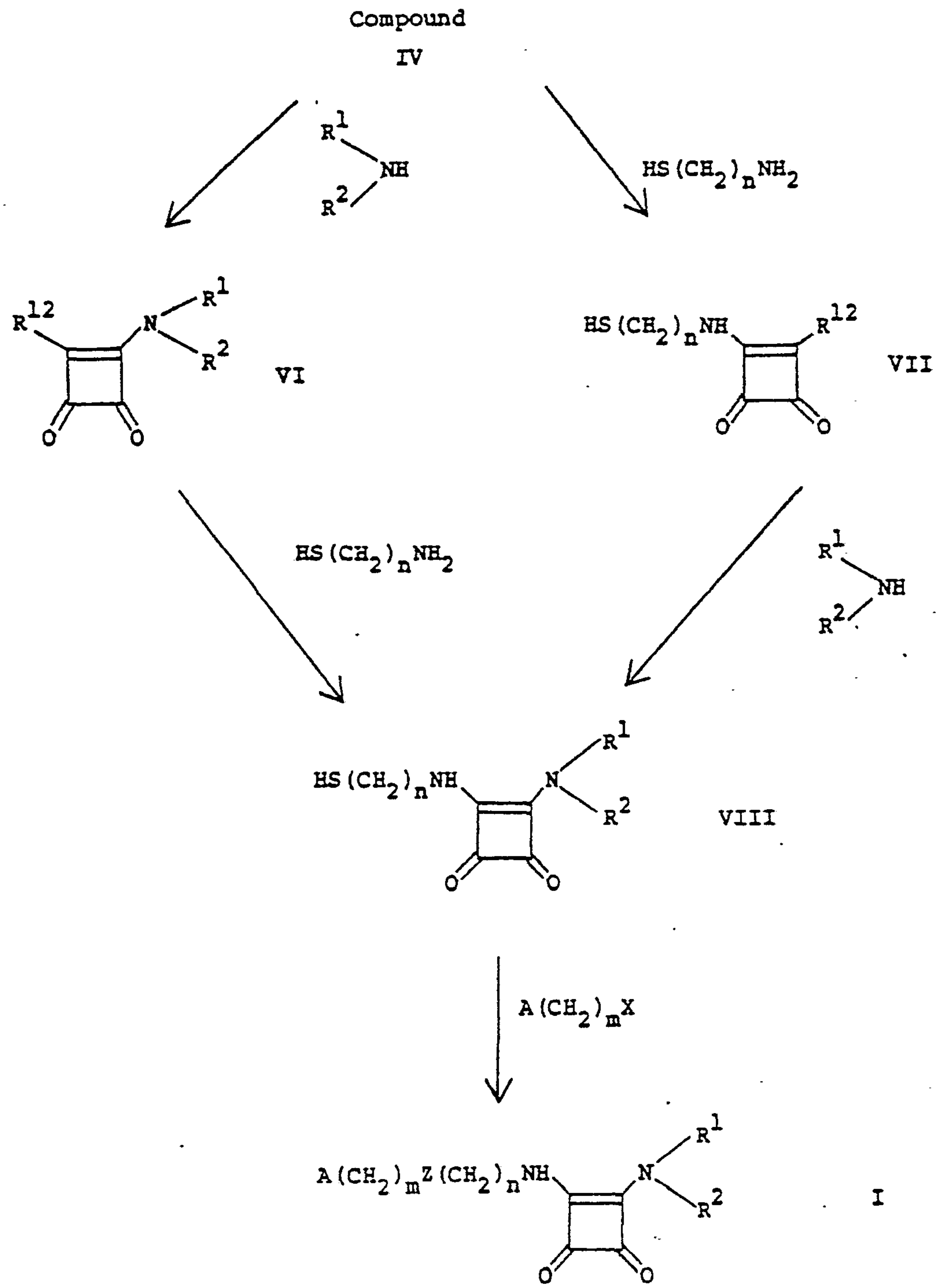


In Reaction Scheme 2, X is a conventional leaving group such as fluoro, chloro, bromo, iodo, $-O_3SR^{13}$ in which $R^{13}$ is $C_1$—$C_6$-alkyl [e.g., methanesulfonate], aryl or substituted aryl [e.g. benzenesulfonate, p-bromobenzenesulfonate or p-toluene-sulfonate), $-O_3SF$, acetoxy or 2,4-dinitrophenoxy. For convenience and economy we prefer to utilize a compound in which X is chloro. The reaction conditions for the preparation of the compounds of Formula VI, VII and VIII are as described for Reaction Scheme 1. The

reaction of the compound of Formula VIII with $A(CH_2)_mX$ may be conducted in any inert organic solvent such as alkanol, acetonitrile, dimethylformamide, dimethylsulfoxide or acetone. Preferably, the reaction is conducted in an alkanol such as methanol, ethanol or isopropanol. The reaction temperature is not critical; the reaction may be conducted at temperatures of from 0° to 200°C. At low temperatures the reaction is slow, while high temperatures normally lead to less pure products due to decomposition and the formation of side-products. Preferably, the reaction is conducted at room temperature. The reaction of the compound of Formula VIII with $A(CH_2)_mX$ to produce the compound of Formula I preferably is conducted in the presence of a base, which facilitates the reaction by acting as an acid acceptor. Suitable bases include, for example, NaOH, NOH, LiOH, triethylamine, dimethylaniline or sodium ethoxide.

In a preferred embodiment, the compounds of Formula I have the structure

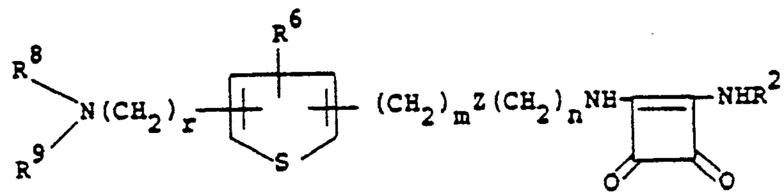

Ia

wherein

$R^2$ is hydrogen, $C_1$—$C_6$-alkyl, allyl, propargyl, 3-pyridylmethyl or 6-methyl-3-pyridylmethyl;
m is an integer of from 0 to 2 inclusive;
n is an integer of from 2 to 5 inclusive;
Z is sulfur, oxygen or methylene;
$R^6$ is hydrogen or $C_1$—$C_6$-alkyl;
r is an integer of from 1 to 4 inclusive; and
$R^8$ and $R^9$ each are independently hydrogen or $C_1$—$C_6$-alkyl, or $R^8$ and $R^9$, taken together with the nitrogen atom to which they are attached, may be pyrrolidin-l-yl, methylpyrrolidin-l-yl, morpholino, thiomorpholino, piperidino, methylpiperidino, 4-methyl-l-piperazinyl, 1,2,3,6-tetrahydropyrid-l-yl, homopiperidino, heptamethyleneimino, octamethyleneimino or 3-azabicyclo[3.2.2]-nonane;
or a nontoxic, pharmaceutically acceptable acid addition salt, hydrate or solvate thereof.

In another preferred embodiment, the compounds of Formula I have the structure

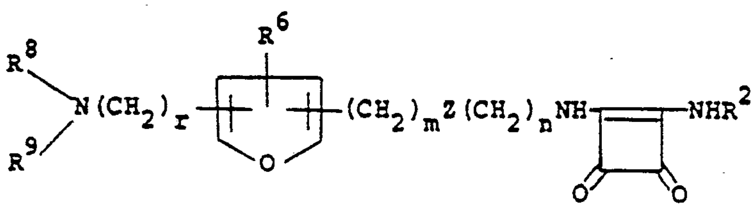

Ib

wherein

$R^2$ is hydrogen, $C_1$—$C_6$-alkyl, allyl, propargyl, 3-pyridylmethyl or 6-methyl-3-pyridylmethyl;
m is an integer of from 0 to 2 inclusive;
n is an integer of from 2 to 5 inclusive;
Z is sulfur, oxygen or methylene;
$R^6$ is hydrogen or $C_1$—$C_6$-alkyl;
r is an integer of from 1 to 4 inclusive; and
$R^8$ and $R^9$ each are independently hydrogen or $C_1$—$C_6$-alkyl, or $R^8$ and $R^9$, taken together with the nitrogen atom to which they are attached, may be pyrrolidin-l-yl, methylpiperidin-l-yl, morpholino, thiomorpholino, piperidino, methylpiperidino, 4-methyl-l-piperazinyl, 1,2,3,6-tetrahydropyrid-l-yl, homopiperidino, heptamethyleneimino, octamethyleneimino or 3-azabicyclo[3.2.2]-nonane;
or a nontoxic, pharmaceutically acceptable acid addition salt, hydrate or solvate thereof.

In another preferred embodiment, the compounds of Formula I have the structure

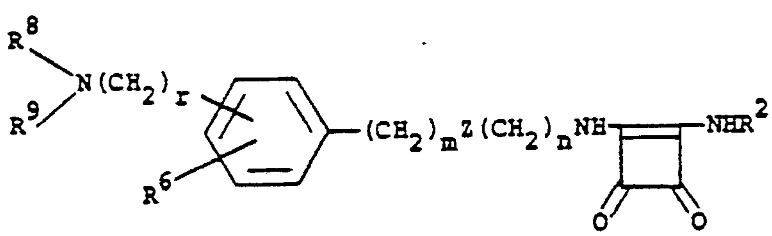

Ic

wherein

$R^2$ is hydrogen or $C_1$—$C_6$-alkyl, allyl, propargyl, 3-pyridylmethyl or 6-methyl-3-pyridylmethyl;
m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-l-yl, methylpyrrolidin-l-yl, morpholino, thiomorpholino, piperidino, methylpiperidino, 4-methyl-l-piperazinyl, 1,2,3,6-tetrahydropyrid-l-yl, homopiperidino, heptamethyleneimino, octamethyleneimino or 3-azabicyclo[3.2.2.]-nonane;

or a nontoxic, pharmaceutically acceptable acid addition salt, hydrate or solvate therof.

In another preferred embodiment, the compounds of Formula I have the structure

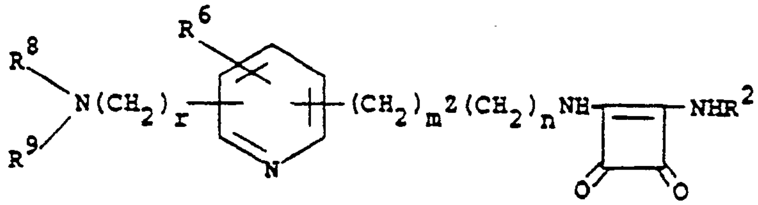

Id

wherein

$R_2$ is hydrogen or $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-l-yl, methylpyrrolidin-l-yl, morpholino, thiomorpholino, piperidino, methylpiperidino, 4-methyl-l-piperazinyl, 1,2,3,6-tetrahydropyrid-l-yl, homopiperidino, heptamethyleneimino, octamethyleneimino or 3-azabicyclo[3.2.2)-nonane;

or a nontoxic, pharmaceutically acceptable acid addition salt, hydrate or solvate thereof.

As presently envisaged, the particularly preferred compounds of Formula I are

a) 1-Amino-2-[3-(3-piperidinomethylphenoxy)propylamino]-cyclobutene-3,4-dione, or a nontoxic pharmaceutically acceptable acid addition salt, hydrate or solvate thereof.

b) 1-Amino-2-[3-(3-piperidinomethylphenoxy)propylamino]-cyclobutene-3,4-dione hydrochloride.

c) 1-Amino-2-{2-[(5-dimethylaminomethyl-2-furyl)-methylthio]ethylamino}cyclobutene-3,4-dione, or a nontoxic pharmaceutically acceptable acid addition salt, hydrate or solvate thereof.

d) 1-Amino-2-{2-[(5-dimethylaminomethyl-3-thienyl)-methylthio]ethylamino}cyclobutene-3,4-dione, or a nontoxic pharmaceutically acceptable acid addition salt, hydrate or solvate thereof.

e) 1-Amino-2-{2-[(5-piperidinomethyl-3-thienyl)methylthio]ethylamino}cyclobutene-3,4-dione, or a nontoxic pharmaceutically acceptable acid addition salt, hydrate or solvate thereof.

f) 1-Amino-2-[3-(3-dimethylaminomethylphenoxy)propylamino]cyclobutene-3,4-dione, or a nontoxic pharmaceutically acceptable acid addition salt, hydrate or solvate thereof.

g) 1-Amino-2-[3-(3-pyrrolidin-l'-ylmethylphenoxy)propylamino]cyclobutene-3,4-dione, or a nontoxic pharmaceutically acceptable acid addition salt, hydrate or solvate thereof.

h) 1-Amino-2-{3-[3-(3-methylpyrrolidin-l'-ylmethyl)phenoxyl]propylamino}cyclobutene-3,4-dione, or a nontoxic pharmaceutically acceptable acid addition salt, hydrate or solvate thereof.

i) 1-Methylamino-2-[3-(3-piperidinomethylphenoxy)-propylamino]cyclobutene-3,4-dione, or a nontoxic pharmaceutically acceptable acid addition salt, hydrate or solvate thereof.

j) 2-[3-(3-Piperidinomethylphenoxy)propylamino]-1-(2-propynylamino)cyclobutene-3,4-dione, or a nontoxic pharmaceutically acceptable acid addition salt, hydrate or solvate thereof.

k) 2-[3-(3-Piperidinomethylphenoxy)propylamino]-1-(3-pyridyl)methylaminocyclobutene-3,4-dione, or a nontoxic pharmaceutically acceptable acid addition salt, hydrate or solvate thereof.

l) 1-Amino-2-[3-(3-hexamethyleneiminomethylphenoxy)-propylamino]cyclobutene-3,4-dione, or a nontoxic pharmaceutically acceptable acid addition salt, hydrate or solvate thereof.

m) 1-Amino-2-[3-(3-piperidinomethylthiophenoxy)propylamino]cyclobutene-3,4-dione, or a nontoxic pharmaceutically acceptable acid addition salt, hydrate or solvate thereof.

n) 1-Amino-2-[3-(3-heptamethyleneiminomethylphenoxy)-propylamino]cyclobutene-3,4-dione, or a nontoxic pharmaceutically acceptable acid addition salt, hydrate or solvate thereof.

o) 1-Amino-2-[3-(3-octamethyleneiminomethylphenoxy)-propylamino]cyclobutene-3,4-dione, or a nontoxic pharmaceutically acceptable acid addition salt, hydrate or solvate thereof.

The starting materials of Formula II used in the preparation of the compounds of this invention are either known or are prepared by methods known in the art. See, for example, the extensive review article by A. H. Schmidt in Synthesis, Pages 961—994 (December 1980) and references cited therein.

Histamine $H_2$-receptor antagonists have been shown to be effective inhibitors of gastric secretion in aminals, including man, Brimblecombe *et al.*, *J. Int. Med. Res.*, *3*, 86 (1975). Clinical evaluation of the histamine $H_2$-receptor antagonist cimetidine has shown it to be an effective therapeutic agent in the treatment of peptic ulcer disease, Gray *et al.*, *Lancet*, *1*, 8001 (1977). Two of the standard animal models for determining gastric antisecretory activity of histamine $H_2$-antagonists are the Gastric Fistula Rat and the Heidenhain Pouch Dog. The $ED_{50}$'s for some of the compounds of Formula I in these two animal models are given in Tables 2 and 3, below.

Determination of Gastric Antisecretory Activity in the Gastric Fistula Rat

Male Long Evans rats weighing about 240—260 grams at the time of cannula implantation are used. The design implantation of the stainless steel cannula into the anterior wall of the fore-stomach are carried out essentially as described by Pare *et al.* (Laboratory Animal Science, *27*, 244 (1977)]. The fistula components are designed and the operative procedure is carried out exactly as described in the above reference. Post operatively the animals are individually housed in solid bottom cages with sawdust and are allowed food and water *ad libitum* throughout the entire recovery period. Animals are not used for test purposes for at least 15 days after the operative procedure.

The animals are fasted but allowed water *ad libitum* for 20 hours before the testing procedure is to begin. Immediately prior to collection, the cannula is opened and the stomach washed gently with 30—40 ml of warm saline or distilled water to remove any residual contents. The catheter is then screwed into the cannula in place of the plugging screw and the rat is placed in a clear plastic rectangular cage measuring 40 cm long, 15 cm wide and 13 cm high. The bottom of the cage has a slit approximately 1.5 cm wide and 25 cm long running down the center to accommodate the catheter which hangs through it. In this way the rat is not restricted and can move freely about the cage during collection periods. The remainder of the assay is carried out as described by Ridley *et al.* [Research Comm. Chem. Path. Pharm., *17*, 365 (1977)].

Gastric secretions collected during the first hour after washing the stomach are discarded as they may be contaminated. For oral evaluation, the catheter is then removed from the cannula and replaced with the plugging screw. Water (2 ml/kg) is administered orally via gastric intubation and the animal is returned to the cage for 45 minutes. After this time the plugging screw is removed and replaced with a catheter to which a small plastic vial has been attached to collect the gastric secretions. A two hour sample is collected (this represents the control secretion), the catheter removed and replaced with the plugging screw. The test drug is now administered orally in a volume of 2 ml/kg via gastric intubation. Forty-five minutes later the plugging screw is again removed, replaced with the catheter attached to a small plastic vial and another 2 hour sample is collected. The secretions in the second sample are compared to those of the control sample in order to determine the effects of the test drug.

When test compounds are to be evaluated parenterally, the animal is injected ip or sc with the test compound vehicle in a volume of 2 ml/kg immediately after discarding the initial 60 minute collection. A two hour sample is collected (control secretion) and the animals are injected either ip or sc with the test compound in a volume of 2 ml/kg. An additional two hour sample is collected and its secretions are compared to those of the control period to determining drug effects.

The samples are centrifuged in a graduated tube for volume determination. Titratable acidity is measured by titrating a one ml sample to pH 7.0 with 0.02N NaOH, using an Autoburet and an electrometric pH meter (Radiometer). Titratable acid output is calculated in microequivalents by multiplying the volume in milliliters by the acid concentration in milliequivalents per liter.

Results are expressed as percent inhibition relative to control readings. Dose response curves are constructed and $ED_{50}$ values are calculated by regression analyses. At least three rats are used at each dosage level and a minimum of three dosage levels are utilized for determination of a dose response curve.

Determination of Gastric Antisecretory Activity in the Heidenhain Pouch Dog

Prior to surgery, hematology and blood chemistry profiles are obtained and an assessment made as to the general health of selected female dogs. Dogs are vaccinated with Tissue Vax 5 (DHLP — Pitman-Moore) and housed in general animal quarters for four weeks' observation so incipient diseases may become apparent. Dogs are fasted with water *ad libitum* 24 hours prior to surgery.

Anesthesia is inducted with Sodium Pentothal (Abbott) 25—30 mg/kg iv. Subsequent anesthesia is maintained with methoxyflurane (Pitman-Moore). A mid-line linea alba incision from xiphoid to umbilicus provides good exposure and ease of closure. The stomach is pulled up into the operative field, the greater curvature stretched out at multiple points and clamps placed along the selected line of incision. The pouch is made from the corpus of the stomach so that true parietal cell juice is obtained. About 30% of the corpus volume is resected. The cannula is made of light-weight, biologically-inert material such as nylon or Delrin® with dimensions and attachments after DeVito and Harkins [J. Appl. Physiol., *14.*, 138 (1959)]. Post operatively, dogs are medicated with antibiotics and an analgesic. They are allowed 2-3 months for recovery. Experiments are carried out in the following way: Dogs are fasted overnight (~18 hours) with water *ad libitum* prior to each experiment. The dogs are placed in a sling and a saphenous vein cannulated for drug administration. Histamine as the base (100 µg/kg/h) and chlorpheniramine maleate (0.25 mg/kg/h) are infused continuously (in a volume of 6 ml/h) with a Harvard infusion pump.

Ninety minutes' infusion are allowed for the dogs to reach a steady state of acid output. At this time the

drug or normal saline (control) is administered concomitantly with the secretagogue in a volume of 0.5 ml/kg over a 30 second period. When oral studies are to be carried out, the drug is administered via gastric gavage in a volume of 5 ml/kg. Infusion of the secretagogue is continued and 15 minute samples of the gastric juice are taken for 4.5 hours. Each sample is measured to the nearest 0.5 ml, and titratable acidity is determined by titrating a 1 ml sample of pH 7.0 with 0.2N NaOH, using an Autoburet and an electrometric pH meter (Radiometer). Titratable acid output is calculated in microequivalents by multiplying the volume in milliliters by the acid concentration in milliequivalents per liter.

Results are expressed as percent inhibition relative to control readings. Dose response curves are constructed and $ED_{50}$ values are calculated by regression and analyses. From 3 toi 5 days are used at each dose level and a minimum of three dosage levels are utilised for determination of a dose response curve.

TABLE 2

Gastric Antisecretory Activity of Compounds of Formula I in the Gastric Fistula Rat

| Compound | $ED_{50}$ sc [μmoles/kg] | Potency Ratio [cimetidine = 1.0] |
|---|---|---|
| Cimetidine | 3.8 (2.3—5.5)* | 1.0 |
| Compound of Example 1 | 0.023 (0.011—0.037) | 162 (77—328)* |
| Compound of Example 2 | <1 | >4 |
| Compound of Example 3 | ~4 | ~1 |
| Compound of Example 9 | 0.055 (0.016—0.14) | 61 (20—204) |
| Compound of Example 10i | 0.08 (0.04—0.16) | 48 (21—97) |
| Compound of Example 10j | 0.044 (0.019—0.09) | 88 (36—211) |
| Compound of Example 10m | 0.7 (0.36—1.4) | 5.4 (2.1—12) |
| Compound of Example 11 | 0.067 (0.02—0.18) | 50 (15—169) |
| Compound of Example 12 | 0.094 (0.046—0.17) | 44 (22—90) |
| Compound of Example 14d | 0.031 (0.016—0.056) | 124 (64—243) |

* numbers in parentheses are 95% confidence limits

TABLE 3

Gastric Antisecretory Activity of Compounds of Formula I in the Heidenhain Pouch Dog

| Compound | $ED_{50}$ [µmoles/kg] | Potency Ratio [cimetidine = 1.0] |
|---|---|---|
| (INTRAVENOUS) | | |
| Cimetidine | 2.18 (1.48—2.95)* | 1.0 |
| Compound of Example 1 | 0.024 (0.019—0.029) | 87 (62—117)* |
| Compound of Example 2 | | ~20 |
| Compound of Example 9 | | ~40 |
| Compound of Example 10i | | >40 |
| (ORAL) | | |
| Cimetidine | 3.29 (1.05—5.19) | 1.0 |
| Compound of Example 1 | 0.16 (0.10—0.22) | 25 (14—40) |
| Compound of Example 9 | | ~20 |

* numbers in parentheses are 95% confidence limits

As used herein and in the claims, alkyl or alkoxy groups preferably contain from 1 to 4 carbon atoms and, most preferably, they contain 1 or 2 carbon atoms. The cycloalkyl rings preferably contain from 3 to 6 carbon atoms.

Celite is a registered trademark of the Johns-Manville Products Corporation for diatomaceous earth.

In the following examples, all temperatures are given in degrees Centigrade.

## Example 1

1-Amino-2-[3-(3-piperidinomethylphenoxy)propylamino]cyclobutene-3,4-dione

A solution of 3-(3-piperidinomethylphenoxy)propylamine (from the dihydrochloride, 4.46 g; 13.9 mmoles) [prepared according to published U.K. Patent Application 2,023,133] in 40 ml of methanol was added all at once to a solution of 1,2-dimethoxycyclobutene-3,4-dione (1.97 g; 13.9 mmoles) in 40 ml of methanol that had been cooled to 5° in an ice-water bath. After 2 hours at ambient temperature, the solution was cooled to 5° and excess anhydrous ammonia was bubbled into the solution for 5 minutes. The mixture was stirred at ambient temperature for 18 hours and then filtered to give 4.35 g of product.

The product (4.20 g; 12.2 mmoles) was suspended in 40 ml of 95% aqueous ethanol and 6.11 ml (12.2 mmoles) of aqueous 2.0N HCl was added with stirring. The solution was filtered through Celite®, cooled at 0° for 17 hours, and then filtered to give 4.33 g of the title compound as its hydrochloride salt, mp 254—257°.

| | | | | |
|---|---|---|---|---|
| *Anal.* Calc'd for $C_{19}H_{26}ClN_3O_3$: | C, 60.08; | H, 6.90; | N, 11.06; | Cl, 9.33. |
| Found (corr. for 0.28% $H_2O$): | C, 59.73; | H, 6.97; | N, 11.14; | Cl, 9.36 |

## Example 2

1-Amino-2-{2-[(5-dimethylaminomethyl-2-furyl)methylthio]ethylamino}cyclobutene-3,4-dione

A solution of 2-[(5-dimethylaminomethyl-2-furyl)methylthio]ethylamine (2.89 g; 13.5 mmoles) [prepared according to the procedure described in Belgian Patent 857,388] in 30 ml of methanol was added dropwise over a period of 30 minutes to a cold (5°) stirred solution of 1,2-dimethoxycyclobutene-3,4-dione (1.92 g; 13.5 mmoles) in 50 ml of methanol. After 3 hours at ambient temperature, the solution was cooled to 5° and excess anhydrous ammonia was bubbled into the solution for 5 minutes. The mixture was stirred at ambient temperature for 18 hours and then filtered to give 2.48 g of the title compound, mp 227—230° (dec.).

An analytical sample was prepared by recrystallization from 95% aqueous ethanol and then from

methanol, and was dried *in vacuo* over $P_2O_5$ for 18 hours to give the title compound as a non-friable sticky solid; the NMR spectrum (100 MHz) in $d_6$ dimethylsulfoxide showed the presence of approximately 0.2 moles of methanol.

*Anal.* Calc'd for $C_{14}H_{19}N_3O_3S{\cdot}0.2\ CH_4O$: C, 54.01; H, 6.32; N, 13.31; S, 10.15.
Found (corr. for 0.54% $H_2O$): C, 53.72; H, 6.07; N, 14.01; S, 10.51

Example 3

1-Amino-2-{2-[(5-dimethylaminomethyl-2-thienyl)methylthio]ethylamino}cyclobutene-3,4-dione

A solution of 2-[(5-dimethylaminomethyl-2-thienyl)methylthio]ethylamine (2.06 g; 8.94 mmoles) [prepared according to the procedure described in Belgian Patent 867,105] in 20 ml of methanol was added all at once to a cold (5°) solution of 1,2-dimethoxycyclobutene-3,4-dione (1.27 g; 8.94 mmoles) in 20 ml of methanol. After 3.5 hours at ambient temperature, the solution was cooled to 5° and excess anhydrous ammonia was bubbled into the soloution for 5 minutes. The mixture was stirredd for 18 hours at ambient temperature and then filtered to give 2.66 g of product. Recrystallization from 95% aqueous ethanol yielded the title compound, mp 240—243° (dec.).

*Anal.* Calc'd for $C_{14}H_{19}N_3O_2S_2$: C, 51.67; H, 5.88; N, 12.91; S, 19.70
Found: C, 51.60; H, 5.76; N, 12.97; S, 19.69

Example 4

2-{2-[(5-Dimethylaminomethyl-2-furyl)methylthio]ethylamino}-1-methylaminocyclobutene-3,4-dione

A solution of 2-[(5-dimethylaminomethyl-2-furyl)methylthio]ethylamine (2.89 g; 13.5 mmoles) in 30 ml of methanol was added all at once to a cold (5°) solution of 1,2-dimethoxycyclobutene-3,4-dione (1.92 g; 13.5 mmoles) in 50 ml of methanol. After 3 hours at ambient temperature, the solution was cooled to 5° and excess anhydrous methylamine was bubbled into the solution for 5 minutes. The mixture was stirred for 18 hours at ambient temperature, evaporated under reduced pressure and then triturated with acetonitrile and filtered to give 2.9 g of crude product. The product was placed on 40 g of silica gel (230—400 mesh) and chromatographed by flash chromatography using a gradient elution of methanol-acetonitrile. The appropriate fractions were evaporated, then combined in methanol, treated with charcoal, filtered and evaporated to near dryness. The solid was triturated with acetonitrile and filtered to give the title compound, mp 176—177.5°.

*Anal.* Calc'd for $C_{15}H_{21}N_3O_3S$: C, 55.71 ;H, 6.54; N, 12.99; S, 9.91
Found (corr. for 1.86% $H_2O$): C, 55.46; H, 6.39; N, 13.14; S, 10.30

Example 5

2-{2-[(5-Dimethylaminomethyl-2-thienyl)methylthio]ethylamino}-1-methylaminocyclobutene-3,4-dione

A solution of 2-[(5-dimethylaminomethyl-2-thienyl)methylthio]ethylamine (1.32 g; 5.73 mmoles) in 20 ml of methanol was added to a cold (5°) solution of 1,2-dimethoxycyclobutene-3,4-dione (814 mg; 5.73 mmoles) in 15 ml of methanol. After 3.5 hours at ambient temperature, the solution was cooled to 5° and excess anhydrous methylamine was bubbled into the solution for 5 minutes. The mixture was stirred for 70 hours at ambient temperature and then filtered to give 1.38 g of product. Recrystallization from ethanol yielded the title compound, mp 185—187°.

*Anal.* Calc'd for $C_{15}H_{21}N_3O_2S_2$: C, 53.07; H, 6.23; N, 12.38; S, 18.89
Found: C, 53.18; H, 6.21; N, 12.25; S, 18.94

Example 6

1-Amino-2-{2-[(5-dimethylaminomethyl-4-methyl-2-thienyl)methylthio]ethylamino}cyclobutene-3,4-dione

A mixture of 2-[(5-dimethylaminomethyl-4-methyl-2-thienyl)methylthio]ethylamine (3.0 g; 12.3 mmoles) [prepared according to the procedure described in published United Kingdom Patent Application 2,063,875] and 1-amino-2-methoxycyclobutene-3,4-dione (1.56 g; 12.3 mmoles) in 50 ml of methanol was stirred at ambient temperature for 18 hours and then filtered to give 3.72 g of product. Recrystallization from 95% aqueous ethanol gave 3.1 g of the title compound.

The product (3.1 g; 9.13 mmoles) was suspended in 40 ml of methanol and 1.52 ml of aqueous 6.0N HCl was added with stirring. The mixture was filtered and the solid was recrystallized from aqueous methanol to give the title compound as its hydrochloride salt, mp 202—205°.

*Anal.* Calc'd for $C_{15}H_{21}N_3O_2S_2HCl$: C, 47.93; H, 5.90; N, 11.18; S, 17.06; Cl, 9.43
Found (corr. for 0.38% $H_2O$): C, 47.74; H, 5.79; N, 11.41; S, 17.21; Cl, 9.42

Example 7

1-Amino-2-{2-[(5-piperidinomethyl-4-methyl-2-thienyl)methylthio]ethylamino}cyclobutene-3,4-dione

The general procedure of Example 6 was repeated except that the 2-[(5-dimethylaminomethyl-4-methyl-2-thienyl)methylthio]ethylamine utilized therein was replaced by an equimolar amount of 2-[(5-piperidinomethyl-4-methyl-2-thienyl)methylthio]ethylamine [prepared according to the procedure described in published United Kingdom Patent Application 2,063,875]. The product (3.64 g; 9.6 mmoles) was suspended in 50 ml of ethanol and 4.8 ml of aqueous 2.0N HCl was added with stirring. The mixture

was filtered and the solid was crystallized from aqueous ethanol to give the title compound as its hydrochloride salt, mp 150—157°.

| | | | | | |
|---|---|---|---|---|---|
| *Anal.* Calc'd for $C_{18}H_{25}N_3O_3S_2HCl$: | C, 51.97; | H, 6.30; | N, 10.10; | S, 15.41; | Cl, 8.52 |
| Found (corr. for 1.58% $H_2O$): | C, 52.05; | H, 6.33; | N, 10.37; | S, 15.24; | Cl, 8.16 |

Example 8

1-Amino-2-[3-(3-piperidinomethylphenoxy)propylamino]cyclobutene-3,4-dione

A solution of 3-(3-piperidinomethylphenoxy)propylamine (from the dihydrochloride, 43.37 g; 0.135 moles) in 250 ml of methanol was added to a suspension of 1-amino-2-methoxycyclobutene-3,4-dione (17.16 g; 0.135 moles) in 350 ml of methanol and stirred at ambient temperature. After 22 hours, the mixture was filtered to give 38.0 g of product.

The product (38.0 g; 0.111 moles) was suspended in 375 ml of aqueous 95% ethanol and 55.3 ml of aqueous 2.0N HCl was added with stirring. The mixture was filtered to give 40.5 g of the title compound as its hydrochloride salt, which is identical to the product prepared in Example 1.

The product was further purified by dissolving in 30% aqueous ethanol then filtered through a pad of silica gel and carbon, evaporated and the solid recrystallized from aqueous ethanol to yield a colorless hydrochloride salt of the title compound, mp 257—259°.

| | | | | |
|---|---|---|---|---|
| *Anal.* Calc'd for $C_{19}H_{25}N_3O_3HCl$: | C, 60.08; | H, 6.90; | N, 11.06; | Cl, 9.33 |
| Found: | C, 59.82; | H, 7.10; | N, 10.87; | Cl, 9.47 |

Example 9

1-Amino-2-[3-(3-dimethylaminomethylphenoxy)propylamino]cyclobutene-3,4-dione

A mixture of 3-(3-dimethylaminomethylphenoxy)propylamine (1.41 g; 6.77 mmoles) [prepared according to the procedure described in Belgian Patent 867,106] and 1-amino-2-methoxycyclobutene-3,4-dione (0.86 g; 6.77 mmoles) in 40 ml of methanol was stirred at ambient temperature for 20 hours and then filtered to give 1.95 g of the title compound.

The product (1.95 g; 6.43 mmoles) was suspended in 35 ml of ethanol and 3.21 ml of 2.0N aqueous HCl was added with stirring. The mixture was filtered and the solid was recrystallized from aqueous ethanol to give the hydrochloride salt of the title compound, mp 205—207°.

| | | | | |
|---|---|---|---|---|
| *Anal.* Calc'd for $C_{16}H_{21}N_3O_3HCl$: | C, 56.55; | H, 6.52; | N, 12.37; | Cl, 10.43 |
| Found: | C, 56.25; | H, 6.56; | N, 12.36; | Cl, 10.27 |

Example 10

The general procedure of Example 9 is repeated, except that the 3-(3-dimethylaminomethylphenoxy)propylamine utilized therein is replaced by an equimolar amount of

(a) 3-(3-pyrrolidin-1-ylmethylphenoxy)propylamine,
(b) 3-[3-(2-methylpyrrolidin-1-ylmethyl)phenoxy]propylamine,
(c) 3-[3-(3-methylpyrrolidin-1-ylmethyl)phenoxy]propylamine,
(d) 3-[3-(4-methylpiperidinomethyl)phenoxy]propylamine,
(e) 3-(3-morpholinomethylphenoxy)propylamine,
(f) 3-[3-(4-hydroxypiperidinomethyl)phenoxy]propylamine,
(g) 3-[3-(4-methyl-1-piperizinylmethyl)phenoxy]propylamine,
(h) 3-[3-(1,2,3,6-tetrahydro-1-pyridylmethyl)phenoxy]propylamine,
(i) 3-(3-hexamethyleneiminomethylphenoxy)propylamine,
(j) 3-(3-heptamethyleneiminomethylphenoxy)propylamine,
(k) 4-(3-piperidinomethylphenoxy)butylamine,
(l) 5-(3-piperidinomethylphenoxy)pentylamine,
(m) 3-(3-octamethyleneiminomethylphenoxy)propylamine,
(n) 3-[3-(3-azabicyclo[3.2.2]non-3-ylmethyl)phenoxy]propylamine and
(o) 3-[3-(3-pyrrolin-1'-ylmethyl)phenoxy]propylamine, respectively,

and there is thereby produced

(a) 1-amino-2-[3-(3-pyrrolidino-1'-ylmethylphenoxy)propylamino]cyclobutene-3,4-dione as its hydrochloride salt, mp 192.5—195°.

| | | | | |
|---|---|---|---|---|
| *Anal.* Calc'd for $C_{18}H_{23}N_3O_3HCl$: | C, 59.10; | H, 6.61; | N, 11.49; | Cl, 9.69 |
| Found (corr. for 0.55% $H_2O$): | C, 58.92; | H, 6.73; | N, 11.61; | Cl, 9.41 |

(b) 1-amino-2-3-[3-(2-methylpyrrolidin-1-ylmethyl)phenoxy]propylaminocyclobutene-3,4-dione as its hydrochloride salt, mp 210—212°.

| | | | | |
|---|---|---|---|---|
| *Anal.* Calc'd for $C_{19}H_{25}N_3O_3HCl$: | C, 60.08; | H, 6.90; | N, 11.06; | Cl, 9.33 |
| Found: | C, 59.97; | H, 6.92; | N, 10.88; | Cl, 9.46 |

(c) 1-amino-2-{3-[3-(3-methylpyrrolidin-1-ylmethyl)phenoxy]propylamino}cyclobutene-3,4-dione as its hydrochloride salt, mp 184.5—187°.

| | | | | |
|---|---|---|---|---|
| *Anal.* Calc'd for $C_{19}H_{25}N_3O_3HCl$: | C, 60.08; | H, 6.90; | N, 11.06; | Cl, 9.33 |
| Found (corr. for 0.26% $H_2O$): | C, 60.43; | H, 7.02; | N, 11.03; | Cl, 9.31 |

(d) 1-amino-2-{3-[3-(4-methylpiperidinomethyl)phenoxy]propylamino}cyclobutene-3,4-dione,
(e) 1-amino-2-[3-(3-morpholinomethylphenoxy)propylamino]cyclobutene-3,4-dione,
(f) 1-amino-2-{3-[3-(4-hydroxypiperidinomethyl)phenoxy]propylamino}cyclobutene-3,4-dione,
(g) 1-amino-2-{3-[3-(4-methyl-1-piperazinylmethyl)phenoxy]propylamino}cyclobutene-3,4-dione,
(h) 1-amino-2-{3-[3-(1,2,3,6-tetrahydro-1-pyridylmethyl)phenoxy]propylamino}cyclobutene-3,4-dione, mp 213—215° (dec.),
(i) 1-amino-2-[3-(3-hexamethyleneiminomethylphenoxy)propylamino]cyclobutene-3,4-dione as its hydrochloride salt, mp 200—202°.

| | | | | |
|---|---|---|---|---|
| *Anal.* Calc'd for $C_{20}H_{27}N_3O_3HCl$: | C, 60.98; | H, 7.16; | N, 10.67; | Cl, 9.00 |
| Found (corr. for 0.28% $H_2O$): | C, 61.25; | H, 7.14; | N, 10.55; | Cl, 8.61 |

(j) 1-amino-2-(3-(3-heptamethyleneiminomethylphenoxy)propylamino]cyclobutene-3,4-dione, mp undefined, gradual decomposition ~200—240°.

| | | | |
|---|---|---|---|
| *Anal.* Calc'd for $C_{21}H_{29}N_3O_3$: | C, 67.90; | H, 7.87; | N, 11.31 |
| Found: | C, 66.44; | H, 7.74; | N, 11.33 |

(k) 1-amino-2-[4-(3-piperidinomethylphenoxy)butylamino]cyclobutene-3,4-dione,
(l) 1-amino-2-[5-(3-piperidinomethylphenoxy)pentylamino]cyclobutene-3,4-dione,
(m) 1-amino-2-[3-(3-octamethyleneiminomethylphenoxy)propylamino]cyclobutene-3,4-dione, mp undefined.

| | | | |
|---|---|---|---|
| *Anal.* Calc'd for $C_{22}H_{31}N_3O_3$: | C, 68.54; | H, 8.11; | N, 10.90 |
| Found: | C, 68.42; | H, 8.42; | N, 11.10 |

(n) 1-amino-2{3-[3-(3-azabicyclo[3.2.2]non-3-ylmethyl)phenoxy]propylamino}cyclobutene-3,4-dione as its hydrochloride salt, mp 162—164° and
(o) 1-amino-2-{3-[3-(3-pyrrolin-1-ylmethyl)phenoxy]propylamino}cyclobutene-3,4-dione, respectively.

## Example 11

1-Methylamino-2-[3-(3-piperidinomethylphenoxy)propylamino]cyclobutene-3,4-dione

A solution of 3-(3-piperidinomethylphenoxy)propylamine (from the dihydrochloride, 3.21 g; 10.0 mmoles) in 40 ml of methanol was added to a solution of 1,2-dimethoxycyclobutene-3,4-dione (1.42 g; 10.0 mmoles) in 40 ml of methanol. After 1 hour at 10° and 30 minutes at ambient temperature, the solution was cooled to 5° and excess anhydrous methylamine was bubbled into the solution for 5 minutes. The mixture was stirred for 17 hours at ambient temperature and then filtered to give 2.77 g of product.

The product (2.77 g) was suspended in 40 ml of ethanol and 4.07 ml (8.1 mmoles) of aqueous 2N HCl was added with stirring to yield the hydrochloride salt of the title compound, mp 194—198°.

| | | | |
|---|---|---|---|
| *Anal.* Calc'd for $C_{20}H_{27}N_3O_3 \cdot HCl$: | C, 60.99; | H, 7.16; | N, 10.67 |
| Found (corr. for 1.35% $H_2O$): | C, 60.63; | H, 6.96; | N, 10.71 |

## Example 12

2-[3-(3-Piperidinomethylphenoxy)propylamino]-1-(3-pyridyl)methylaminocyclobutene-3,4-dione

The general procedure of Example 11 was repeated, except that the methylamine utilized therein was replaced by 1.08 g (10.0 mmoles) of 3-aminomethylpyridine. The crude product was placed on 65 g of silica gel (230—400 mesh) and chromatographed by flash chromatography using a gradient elution of methanolmethylene chloride containing 1% $NH_4OH$. The appropriate fractions were combined, evaporated and the solid residue recrystallized from methanol to give the title compound, mp 174—178.5°.

| | | | |
|---|---|---|---|
| *Anal.* Calc'd for $C_{25}H_{30}N_4O_3$: | C, 69.10; | H, 6.96; | N, 12.89 |
| Found (corr. for 0.52% $H_2O$): | C, 68.80; | H, 7.03; | N, 12.74 |

## Example 13

2-[3-(3-Piperidinomethylphenoxy)propylamino]-1-propylaminocyclobutene-3,4-dione

The general procedure of Example 11 was repeated, except that the methylamine utilized therein was replaced by 4.0 ml (48.7 mmoles) of propylamine. The crude product was placed on 60 g of silica gel (230—400 mesh) and chromatographed by flash chromatography using a gradient elution of methanol-methylene chloride. The appropriate fractions were combined and the solid residue recrystallized from methanol to yield the title compound, mp 158—160°.

| | | | |
|---|---|---|---|
| *Anal.* Calc'd for $C_{22}H_{31}N_3O_3$: | C, 68.54; | H, 8.11; | N, 10.90 |
| Found: | C, 68.11; | H, 8.25; | N, 11.21 |

## Example 14

The general procedure of Example 12 is repeated, except that the 3-aminomethylpyridine utilized therein is replaced by an excess molar amount of
(a) ethylamine,
(b) *n*-butylamine,
(c) allylamine,

(d) propargylamine,
(e) benzylamine and
(f) 6-methyl-3-aminomethylpyridine, respectively,
and there is thereby produced
(a) 1-ethylamino-2-[3-(3-piperidinomethylphenoxy)propylamino]cyclobutene-3,4-dione,
(b) 1-butylamino-2-[3-(3-piperidinomethylphenoxy)propylamino]cyclobutene-3,4-dione,
(c) 1-allylamino-2-[3-(3-piperidinomethylphenoxy)propylamino]cyclobutene-3,4-dione, mp 158—159.5°.

| | | | |
|---|---|---|---|
| *Anal.* Calc'd for $C_{22}H_{29}N_3O_3$: | C, 68.90; | H, 7.62; | N, 10.96 |
| Found: | C, 68.81; | H, 7.70; | N, 10.72 |

(d) 2-[3-(3-piperidinomethylphenoxy)propylamino]-1-(2-propynylamino)cyclobutene-3,4-dione, mp 158—160°.

| | | | |
|---|---|---|---|
| *Anal.* Calc'd for $C_{22}H_{27}N_3O_3$: | C, 69.27; | H, 7.13; | N, 11.02 |
| Found: | C, 69.26; | H, 7.25; | N, 10.78 |

(e) 1-benzylamino-2-[3-(3-piperidinomethylphenoxy)propylamino]cyclobutene-3,4-dione hydrochloride, mp 136—140°.

| | | | | |
|---|---|---|---|---|
| *Anal.* Calc'd for $C_{26}H_{31}N_3O_3HCl$: | C, 66.44; | H, 6.86; | N, 8.94; | Cl, 7.54 |
| Found: | C, 65.41; | H, 7.08; | N, 8.83; | Cl, 7.67 |

and
(f) 1-(6-methyl-3-pyridyl)methylamino-2-[3-(3-piperidinomethylphenoxy)propylamino]cyclobutene-3,4-dione, respectively.

## Example 15

1-Amino-2-[3-(6-piperidinomethyl-2-pyridyloxy)propylamino]cyclobutene-3,4-dione

A. 2-Chloro-6-piperidinomethylpyridine

To 2-chloro-6-methylpyridine (50.0 g, 0.392 mole) in 393 ml of carbon tetrachloride was added N-bromosuccinimide (87.2 g, 0.49 mole) and 1.0 g of benzoyl peroxide. The mixture was stirred at reflux for 22 hours, cooled to 10° and filtered. The chilled filtrate then was treated slowly with piperidine (83.5 g, 0.98 mole) and allowed to stir at ambient temperature for 18 hours. After removal of the piperidine hydrobromide by filtration, the filtrate was concentrated to about half volume and extracted with 6N HCl (65 ml) and 3N HCl (40 ml). The acid extracts were made basic with 40% sodium hydroxide and the product was extracted into methylene chloride. The solvent was evaporated and the residue distilled to yield 41% of the title compound as a colorless oil, bp 101—103°/60 Pa (0.45 mmHg).

| | | | | |
|---|---|---|---|---|
| *Anal.* Calc'd for $C_{11}H_{15}ClN_2$: | C, 62.71; | H, 7.18; | 13.29; | Cl, 16.82 |
| Found: | C, 61.71; | H, 7.31; | 13.63; | C, 17.20 |

B. N-[3-(6-Piperidinomethyl-2-pyridyloxy)propyl]formamide

3-Aminopropanol (12.84 g, 0.171 mole) was added to a suspension of 50% sodium hydride in mineral oil (7.96 g, 0.166 mole) in 180 ml of dry DMF and the mixture was warmed to 80—83°. A solution of 2-chloro-6-piperidinomethylpyridine (34.0 g, 0.161 mole) [prepared in Step A] in 180 ml of dry DMF was then added dropwise and when complete, the temperature was raised to 125—128° for 3 hours followed by 17 hours at ambient temperature. The precipitated salts were removed by filtration and the solvent stripped under vacuum. The oily residue was dissolved in methylene chloride, washed with water, dried and the solvent evaporated. This residue was redissolved in acetonitrile and extracted with skelly B. After removing the solvent the crude oil was purified by flash chromatography on 270 g of silica gel (230—400 mesh) using a gradient elution of methanol-methylene chloride and evaporated to give the title product as a yellow oil, 21.63 g (48.4%).

C. 3-(6-Piperidinomethyl-2-pyridyloxy)propylamine

N-[3-(6-Piperidinomethyl-2-pyridyloxy)propyl]formamide (19.6 g, 70.7 mmoles) [prepared in Step B] was added to a solution of 85% potassium hydroxide pellets (18.63 g, 0.332 mole) dissolved in 180 ml of methanol and the solution was heated at gentle reflux for 20 hours. The solvent was stripped in vacuum and the residue partially purified by redissolving in about 180 ml of 20% methanol-methylene chloride and passing through a pad of 38 g of silica gel. The silica was washed with an additional 120 ml of eluant and the combined filtrates were evaporated to an amber oil. Final purification was effected by flash chromatography on 120 g of silica gel (230—400 mesh) using a gradient elution of methanol-methylene chloride containing 0.5% $NH_4OH$. The title compound was obtained as a yellow oil in 63% yield.

D. 1-Amino-2-[3-(6-piperidinomethyl-2-pyridyloxy)propylamino]cyclobutene-3,4-dione

A mixture of 3-(6-piperidinomethyl-2-pyridyloxy)propylamine (2.5 g, 10 mmoles) [prepared in Step C] and 1-amino-2-methoxycyclobutene-3,4-dione (1.27 g, 10 mmoles) in 35 ml of methanol was stirred at ambient temperature for 18 hours and filtered to give 2.71 g of product.

The product (2.71 g, 7.87 mmoles) was suspended in 35 ml of absolute ethanol and 7.87 ml of aqueous 6.0N HCl was added with stirring. After 64 hours at 0° the salt was collected by filtration and recrystallized from aqueous ethanol to give the hydrochloride salt of the title compound, mp 255—258°.

| | | | | |
|---|---|---|---|---|
| *Anal.* Calc'd for $C_{18}H_{24}N_4O_3HCl$: | C, 56.77; | H, 6.61; | N, 14.71; | Cl, 9.31 |
| Found: | C, 56.71; | H, 6.80; | N, 14.41; | Cl, 9.98 |

Example 16

1-Amino-2-[3-(6-dimethylaminomethyl-2-pyridyloxy)propylamino]cyclobutene-3,4-dione

The general procedure of Example 15 was repeated, except that the piperidine utilized in Step A was replaced with an excess of anhydrous dimethlamine. The product (2.26 g, 7.43 mmoles) was suspended in 40 ml of 95% ethanol and 7.43 ml of aqueous 2.0N HCl was added with stirring. After evaporating most of the solvent, the residue was triturated under isopropyl alcohol and recrystallized from aqueous ethanol to give the hydrochloride salt of the title compound, mp 230—234° (dec.).

| | | | | |
|---|---|---|---|---|
| *Anal.* Calc'd for $C_{15}H_{20}N_4O_3HCl$: | C, 52.87; | H, 6.21; | N, 16.44; | Cl, 10.40 |
| Found: | C, 51.52; | H, 5.98; | N, 16.64; | Cl, 10.88 |

Example 17

1-Amino-2-[2-(3-piperidinomethylthiophenoxy)ethylamino]cyclobutene-3,4-dione

A mixture of m-dithiobenzoic acid (20.8 g, 67.9 mmoles) [prepared according to the procedure described in *J. Chem. Soc.,* London, *119,* 1792 (1921)] and thionyl chloride (200 ml) was refluxed for four hours, filtered and the excess $SOCl_2$ removed in vacuum.

B. Dithio bis-3,3′-N,N-di(piperidino)benzenecarboxamide

The crude product from Step A, dissolved in 100 ml of tetrahydrofuran, was added dropwise at 3° to a solution of piperidine (25.1 g, 0.29 mole) in 500 ml of tetrahydrofuran. The mixture was stirred at ambient temperature for 76 hours and poured into 1500 ml of dilute HCl (ca. 2N). After one hour the product was extracted into ether and washed sequentially with water, aqueous 1N NaOH and water. The solvent was evaporated to leave 26.4 g of the title compoune.

C. 3-(Piperidinomethyl)thiophenol

To a suspension of lithium aluminum hydride (45.3 g, 1.19 moles) in 2200 ml of ether was added, dropwise under nitrogen, a solution of dithio bis-3,3′-N,N-di(piperidino)benzenecarboxamide (141.5 g, 0.32 mole) [prepared in Step B] in 2200 ml of ether and the mixture was stirred at ambient temperature for 20 hours. The mixture was decomposed by the addition of saturated sodium sulfate solution and filtered. The filter cake was stirred with 3000 ml of water and a solution of citric acid monohydrate (550 g, 2.62 moles) in 550 ml of water was added. The pH of the solution was adjusted to about 2 with 12N HCl and then to pH 8 with concentrated ammonium hydroxide. The solution was exhaustively extracted with ether to yield 120 g of product.

An aliquot of the title compound was recrystallized from isopropyl alcohol, mp 121—123°; Mass spectrum 206 ($M^+$).

| | | | | |
|---|---|---|---|---|
| *Anal.* Calc'd for $C_{12}H_{17}NS$: | C, 69.56; | H, 8.21; | N, 6.76; | S, 15.46 |
| Found: | C, 69.02; | H, 8.03; | N, 6.67; | S, 15.06 |

D. N-{2-[3-(Piperidinomethyl)thiophenoxy]ethyl}phthalimide

A mixture of 3-(piperidinomethyl)thiophenol (2.07 g, 10 mmoles) [prepared in Step C] and N-(2-bromoethyl)phthalimide (2.41 g, 9.5 mmoles) in 10 ml of dry DMF was stirred at ambient temperature for 84 hours. The solvent was evaporated under reduced pressure and the crude oil flask chromatographed on 100 g of silica gel (230—400 mesh) using 2.5% methanol in methylene chloride with 0.2% $NH_4OH$ as the eluant. The appropriate fractions were combined and evaporated to give an oil that crystallized under ether. Recrystallization from acetonitrile yielded 1.2 g of the hydrobromide salt of the title compound, mp 180—181.5°.

| | | | | | |
|---|---|---|---|---|---|
| *Anal.* Calc'd for $C_{22}H_{24}N_2O_2SHBr$: | C, 57.26; | H, 5.46; | N, 6.07; | Br, 17.32; | S, 6.95 |
| Found: | C, 56.98; | H, 5.43; | N, 6.30; | Br, 17.51; | S, 7.10 |

E. 2-(3-Piperidinomethylthiophenoxy)ethylamine

Anhydrous hydrazine (1.79 g, 56.0 mmoles) was added to a suspension of N-{2-[3-(piperidinomethyl)thiophenoxy]ethyl}phthalimide hydrobromide (5.17 g, 11.2 mmoles) [prepared in Step D] in 200 ml of 95% ethanol, stirred at ambient temperature for 18 hours and filtered. The filtrate was stripped and the semi-solid residue was stirred with several portions of ether. Evaporation of the solvent gave 2.8 g of title compound as a yellow oil.

F. 1-Amino-2-[2-(3-piperidinomethylthiophenoxy)ethylamino]cyclobutene-3,4-dione

The crude amine prepared in Step E (1.4 g, 4.05 mmoles) in 40 ml of methanol was added to a suspension of 1-amino-2-methoxycyclobutene-3,4-dione (0.515 g, 4.05 mmoles) in 100 ml of methanol. The mixture was stirred for 20 hours at ambient temperature and then filtered to give 0.8136 g of product. A

second crop was obtained from the concentrated mother liquor and the combined lots were recrystallized from methanol to yield 0.786 g (56%) of the title compound, mp 228—230° (dec.).

| | | | | |
|---|---|---|---|---|
| *Anal.* Calc'd for $C_{18}H_{23}N_3O_2S$: | C, 62.58; | H, 6.71; | N, 12.16; | S, 9.28 |
| Found: | C, 62.17; | H, 6.36; | N, 12.59; | S, 9.60 |

Example 18

1-Amino-2-[3-(3-piperidinomethylthiophenoxy)propylamino]cyclobutene-3,4-dione

A. N-{3-[3-(Piperidinomethyl)thiophenoxy]propyl}phthalimide

The general procedure of Example 17, Step D, was repeated, except the N-(2-bromoethyl)phthalimide utilized therein was replaced with an equimolar amount of N-(3-bromopropyl)phthalimide. The chromatographed product was recrystallized from isopropyl alcohol to give the title compound as its hydrobromide salt, mp 188—192°.

| | | | | |
|---|---|---|---|---|
| *Anal.* Calc'd for $C_{23}H_{26}N_2O_2SHBr$: | C, 58.10; | H, 5.72; | N, 5.89; | Br, 16.81 |
| Found: | C, 57.79; | H, 5.41; | N, 5.73; | Br, 16.80 |

B. 3-(3-Piperidinomethylthiophenoxy)propylamine

To a solution of N-{3-[3-(piperidinomethyl)thiophenoxy]propyl}phthalimide hydrobromide (58.0 g, 0.12 mole) [prepared in Step A] in 1650 ml of 95% ethanol was added hydrazine hydrate (26.9 g, 0.54 mole) and the reaction mixture was heated at 45° for 4.5 hours. The mixture was diluted with 500 ml of ether, filtered and the filtrate evaporated to dryness to give the title compound as an amber oil (14.1 g). An aliquot was distilled to a colorless oil, bp 154—155°/20 Pa (0.15 mmHg).

| | | | |
|---|---|---|---|
| *Anal.* Calc'd for $C_{15}H_{24}N_2S$: | C, 68.13; | H, 9.15; | N, 10.59 |
| Found: | C, 67.37; | H, 9.07; | N, 10.94 |

C. 1-Amino-2-[3-(3-piperidinomethylthiophenoxy)propylamino]cyclobutene-3,4-dione

1-Amino-2-methoxycyclobutene-3,4-dione (1.20 g, 9.5 mmoles) was added to a solution of the crude amine prepared in Step B (2.50 g, 9.5 mmoles) in 75 ml of methanol and the mixture was stirred at ambient temperature for 16 hours. The precipitate was filtered to give 2.82 g of crude product.

The crude solid (2.82 g, 7.84 mmoles) was suspended in 30 ml of 95% ethanol and 4.0 ml of aqueous 2.0N HCl was added with stirring. After ca. 15 minutes 40 ml of acetone was added and the mixture stored at ambient temperature for 16 hours. The precipitate was recrystallized from aqueous ethanol to yield 1.64 g of the hydrochloride salt of the title compound, mp 236—237.5°.

| | | | | | |
|---|---|---|---|---|---|
| *Anal.* Calc'd for $C_{19}H_{25}N_3O_3S{\cdot}HCl$: | C, 57.64; | H, 6.62; | N, 10.61; | S, 8.10; | Cl, 8.95 |
| Found: | C, 57.72; | H, 6.56; | N, 10.66; | S, 8.49; | Cl, 8.88 |

Example 19

1-Amino-2-{2-[(5-dimethylaminomethyl-3-thienyl)methylthio]ethylamino}cyclobutene-3,4-dione

A mixture of 2-[(5-dimethylaminomethyl-3-thienyl)methylthio]ethylamine (2.11 g, 8.68 mmoles) [prepared according to the procedure described in published European Patent Application 27,744] and 1-amino-2-methoxycyclobutene-3,4-dione (1.10 g, 8.68 mmoles; in methanol was stirred at ambient temperature for 18 hours and filtered. The crude product was recrystallized from 2-methoxyethanol to yield 1.30 g of the title compound as a colorless solid, mp 234—236°.

| | | | | |
|---|---|---|---|---|
| *Anal.* Calc'd for $C_{14}H_{19}N_3O_2S_2$: | C, 51.66; | H, 5.88; | N, 12.91; | S, 19.71 |
| Found: | C, 51.53; | H, 5.64; | N, 12.62; | S, 19.91 |

Example 20

1-Amino-2-{2-[(5-piperidinomethyl-3-thienyl)methylthio]ethylamino}cyclobutene-3,4-dione

The general procedure of Example 19 was repeated except that the 2-[(5-dimethylaminomethyl-3-thienyl)methylthio]ethylamine utilized therein was replaced by an equimolar amount of 2-[(5-piperidinomethyl-3-thienyl)methylthio]ethylamine [prepared according to the procedure described in published Eurolpean Patent Application 27,744]. The crude solid was recrystallized from 2-methoxyethanol to give the title compound (1.27 g), mp 236—238°.

| | | | | |
|---|---|---|---|---|
| *Anal.* Calc'd for $C_{17}H_{23}N_3O_2S_2$: | C, 55.86; | H, 6.34; | N, 11.50; | S, 17.54 |
| Found: | C, 55.59; | H, 6.23; | N, 11.75; | S, 17.62 |

Example 21

1-Amino-2-[3-(5-dimethylaminomethyl-3-thienyloxy)propylamino]cyclobutene-3,4-dione

An equimolar mixture of 4-[3-(amino)propoxy]-N,N-dimethyl-2-thiophenemethanamine [prepared according to the procedure described in published European Patent Application 27,744] and 1-amino-2-methoxycyclobutene-3,4-dione is reacted according to the general procedure of Example 19 to yield the title compound.

**Claims for the Contracting States: BE CH DE FR GB IT LI LU NL SE**

1. A pharmaceutical composition useful in the treatment of peptic ulcers which comprises a peptic activity-inhibiting amount of pepstatin and an effective anti-ulcerogenic amount of at least one compound of the formula I:

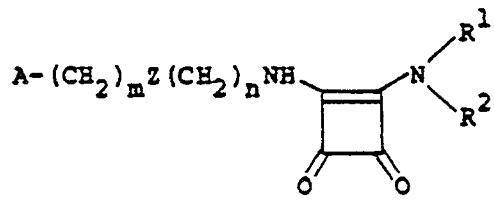

I

wherein $R^1$ and $R^2$ each are independently hydrogen or $C_1$—$C_6$-alkyl, and, when $R^1$ is hydrogen, $R^2$ also may be allyl, propargyl, cyclo-$C_3$—$C_7$-alkyl $C_1$—$C_6$-alkyl, cyclo-$C_3$—$C_7$-alkyl, cyano-$C_1$—$C_6$-alkyl, 2-fluoroethyl, 2,2,2-trifluoroethyl, hydroxy, 2,3-dihydroxypropyl,

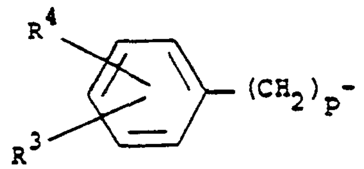

or

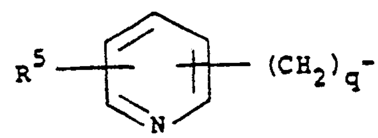

in which

p is an integer of from 1 to 6 inclusive;

q is an integer of from 1 to 6 inclusive;

$R^3$ and $R^4$ each are independently hydrogen, $C_1$—$C_6$-alkyl, hydroxy, $C_1$—$C_6$-alkoxy or halogen, and, when $R^3$ is hydrogen, $R^4$ also may be trifluoromethyl, or $R^3$ and $R^4$, taken together, may be methylenedioxy, $R^5$ is hydrogen;

m is an integer of from 0 to 2 inclusive;

n is an integer of from 2 to 5 inclusive;

Z is sulfur, oxygen or methylene; and

A is

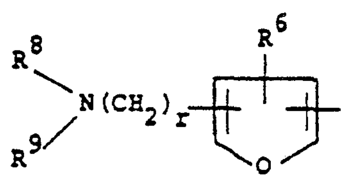

,

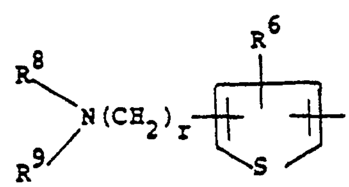

,

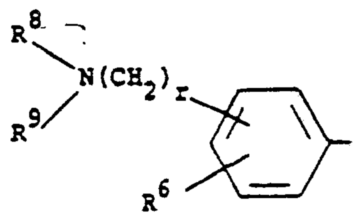

or

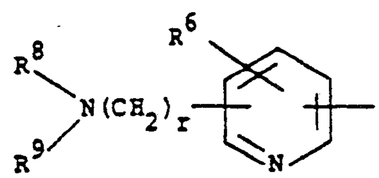

in which

$R^6$ is hydrogen, $C_1$—$C_6$-alkyl, $C_1$—$C_6$-alkoxy or halogen;

r is an integer of from 1 to 4 inclusive; and

$R^8$ and $R^9$ each are independently hydrogen, $C_1$—$C_6$-alkyl, allyl, propargyl, $C_1$—$C_6$-alkoxy-$C_1$—$C_6$-alkyl in which the $C_1$—$C_6$-alkoxy moiety is at least two carbon atoms removed from the nitrogen atom, cyclo-$C_3$—$C_7$-alkyl, or phenyl-$C_1$—$C_6$-alkyl, provided that $R_8$ and $R_9$ may not both be cyclo-$C_3$—$C_7$-alkyl, or $R^8$ and $R^9$, taken together with the nitrogen atom to which they are attached, may be pyrrolidin-1-yl, methylpyrrolidin-1-yl, dimethylpyrrolidin-1-yl, morpholino, thiomorpholino, piperidino, methylpiperidino, dimethylpiperidino, hydroxypiperidino, 4-methyl-1-piperazinyl, 1,2,3,6-tetrahydropyrid-1-yl, 3-pyrrolin-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

2. A composition of Claim 1 wherein $R^1$ and $R^2$ each are independently hydrogen or $C_1$—$C_6$-alkyl, and,

when $R^1$ is hydrogen, $R^2$ also may be allyl, propargyl, $C_3$—$C_7$-cycloalkyl-$C_1$—$C_6$-alkyl,

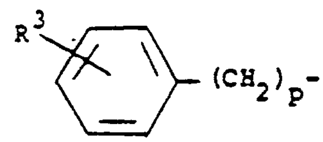

or

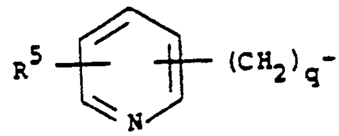

in which

p and q each are independently an integer of from 1 to 6 inclusive and $R^3$ and $R^5$ each are hydrogen, $C_1$—$C_6$-alkyl or $C_1$—$C_6$-alkoxy;

m is an integer of from 0 to 2 inclusive;

n is an integer of from 2 to 5 inclusive;

Z is sulfur, oxygen or methylene; and

A is

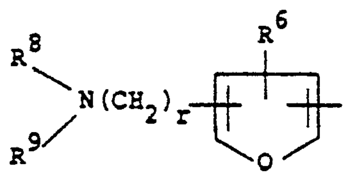

,

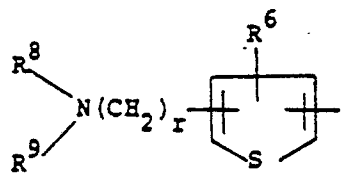

,

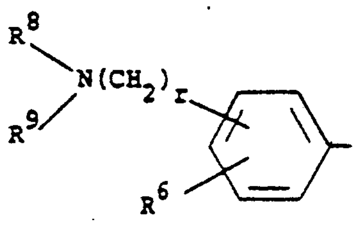

or

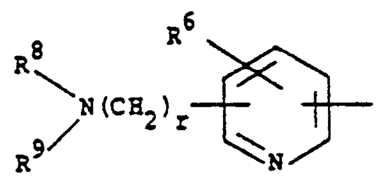

in which

$R^6$ is hydrogen, $C_1$—$C_6$-alkyl or $C_1$—$C_6$-alkoxy;

r is an integer of from 1 to 4 inclusive; and

$R^8$ and $R^9$ each are independently hydrogen or $C_1$—$C_6$-alkyl, or, $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

3. A composition of Claim 1 wherein the compound of Formula I has the structure

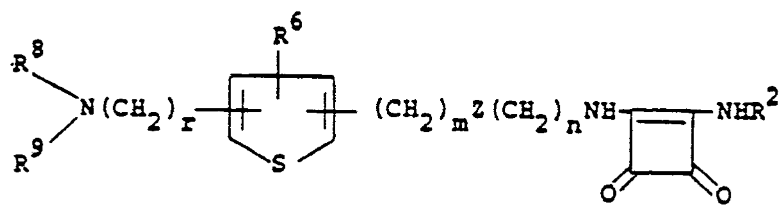

Ia

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

4. A composition of Claim 1 wherein the compound of Formula I has the structure

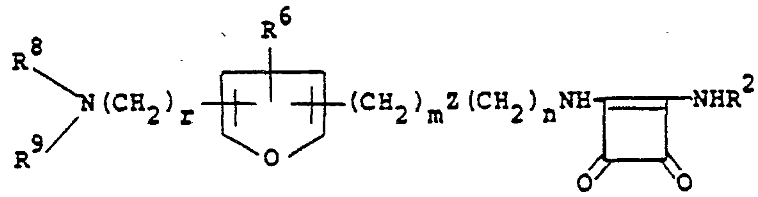

Ib

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

5. A composition of Claim 1 wherein the compound of Formula I has the structure

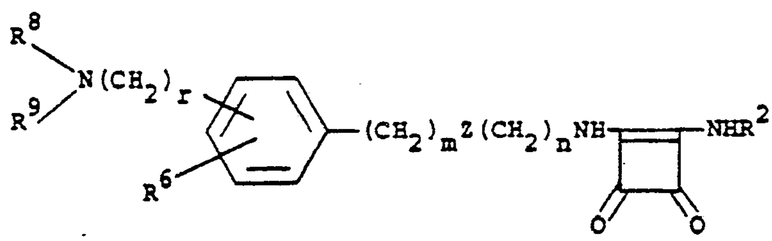

Ic

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

6. A composition of Claim 1 wherein the compound of Formula I has the structure

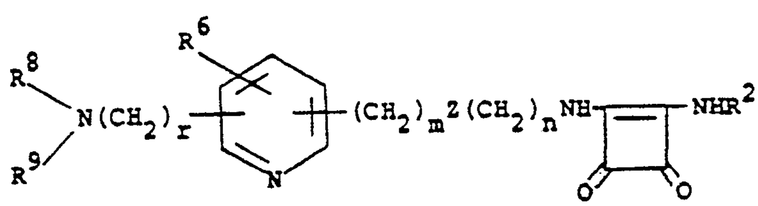

Id

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl,

homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

7. A composition of Claim 5 wherein $R^2$ is hydrogên.

8. A composition of any one of Claims 1—7, wherein the dosage of pepstatin is from 100 to 175 mg and the dosage of the compound of Formula I is from 2 to 100 mg.

9. A composition of claim 8 wherein the dosage of the compound of Formula I is from 4 to 50 mg.

10. A pharmaceutical composition of claim 8 which comprises from 100 to 175 mg of pepstatin and from 2 to 15 mg of 1-amino-2-[3-(3-piperidinomethylphenoxy)propylamino]-cyclobutene-3,4-dione or a nontoxic, pharmaceutically acceptable acid addition salt, hydrate or solvate thereof; and a pharmaceutically acceptable carrier.

11. A pharmaceutical composition of claim 8 which comprises from 100 to 175 mg of pepstatin and from 2 to 15 mg of 1-amino-2-[3-(3-dimethylaminomethylphenoxy)-propylamino]cyclobutene-3,4-dione, or a nontoxic, pharmaceutically acceptable acid addition salt, hydrate or solvate thereof; and a pharmaceutically acceptable carrier.

12. A composition of claims 8—11 in which the pepstatin is in the form of pepstatin floating minicapsules.

13. A composition of any one of claims 1—12 in unit dosage form.

**Claims for the Contracting State: AT**

1. A process for preparing a pharmaceutical composition useful in the treatment of peptic ulcers which comprises mixing a peptic activity-inhibiting amount of pepstatin and an effective anti-ulcerogenic amount of at least one compound of the formula I:

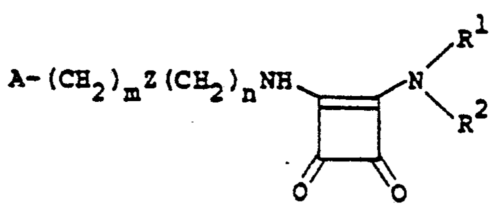

I

wherein $R^1$ and $R^2$ each are independently hydrogen or $C_1$—$C_6$-alkyl, and, when $R^1$ is hydrogen, $R^2$ also may be allyl, propargyl, cyclo-$C_3$—$C_7$-alkyl $C_1$—$C_6$-alkyl, cyclo-$C_3$—$C_7$-alkyl, cyano-$C_1$—$C_6$-alkyl, 2-fluoroethyl, 2,2,2-trifluoroethyl, hydroxy, 2,3-dihydroxypropyl,

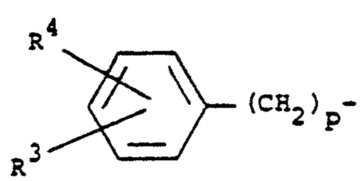

or

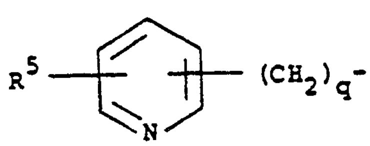

in which

p is an integer of from 1 to 6 inclusive;

q is an integer of from 1 to 6 inclusive;

$R^3$ and $R^4$ each are independently hydrogen, $C_1$—$C_6$-alkyl, hydroxy, $C_1$—$C_6$-alkoxy or halogen, and, when $R^3$ is hydrogen, $R^4$ also may be trifluoromethyl, or $R^3$ and $R^4$, taken together, may be methylenedioxy, $R^5$ is hydrogen;

m is an integer of from 0 to 2 inclusive;

n is an integer of from 2 to 5 inclusive;

Z is sulfur, oxygen or methylene; and

A is

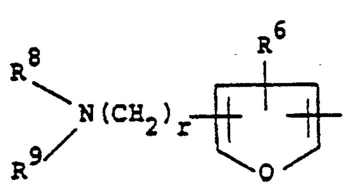

,

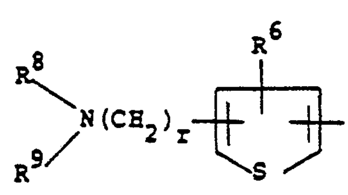

,

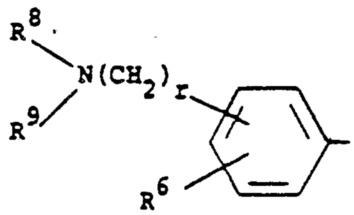

or

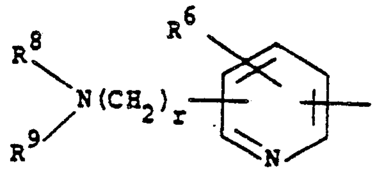

in which

$R^6$ is hydrogen, $C_1$—$C_6$-alkyl, $C_1$—$C_6$-alkoxy or halogen;

r is an integer of from 1 to 4 inclusive; and

$R^8$ and $R^9$ each are independently hydrogen, $C_1$—$C_6$-alkyl, allyl, propargyl, $C_1$—$C_6$-alkoxy-$C_1$—$C_6$-alkyl in which the $C_1$—$C_6$-alkoxy moiety is at least two carbon atoms removed from the nitrogen atom, cyclo-$C_3$—$C_6$-alkyl, or phenyl-$C_1$—$C_6$-alkyl, provided that $R^8$ and $R^9$ may not both be cyclo-$C_3$—$C_7$-alkyl, or $R^8$ and $R^9$, taken together with the nitrogen atom to which they are attached, may be pyrrolidin-1-yl, methylpyrrolidin-1-yl, dimethylpyrrolidin-1-yl, morpholino, thiomorpholino, piperidino, methylpiperidino, dimethylpiperidino, hydroxypiperidino, 4-methyl-1-piperazinyl, 1,2,3,6-tetrahydropyrid-1-yl, 3-pyrrolin-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

2. A process of Claim 1 wherein $R^1$ and $R^2$ each are independently hydrogen or $C_1$—$C_6$-alkyl, and, when $R^1$ is hydrogen, $R^2$ also may be allyl, propargyl, cyclo-$C_3$—$C_7$-alkyl, —$C_1$—$C_6$-alkyl,

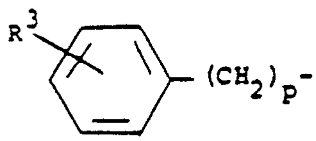

or

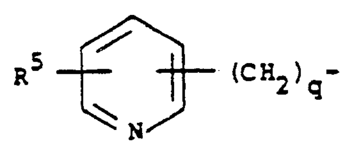

in which

p and q each are independently an integer of form 1 to 6 inclusive and $R^3$ and $R^5$ each are hydrogen, $C_1$—$C_6$-alkyl or $C_1$—$C_6$-alkoxy;

m is an integer of from 0 to 2 inclusive;

n is an integer of from 2 to 5 inclusive;

Z is sulfur, oxygen or methylene; and

A is

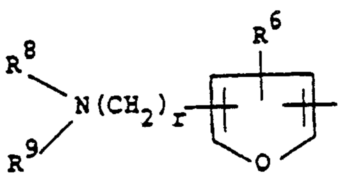

,

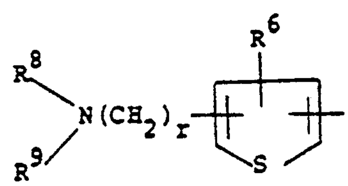

,

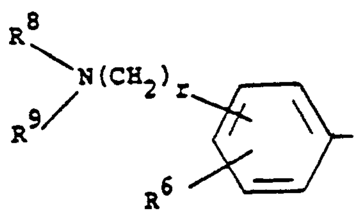

or

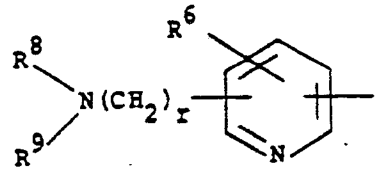

in which

$R^6$ is hydrogen, $C_1$—$C_6$-alkyl or $C_1$—$C_6$-alkoxy;

r is an integer of from 1 to 4 inclusive; and

$R^8$ and $R^9$ each are independently hydrogen or $C_1$—$C_6$-alkyl, or, $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

3. A process of Claim 1 wherein the compound of Formula I has the structure

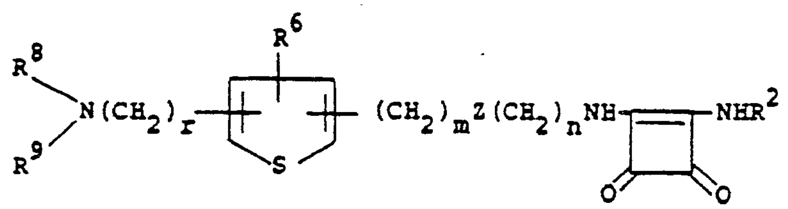

Ia

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

4. A process of Claim 1 wherein the compound of Formula I has the structure

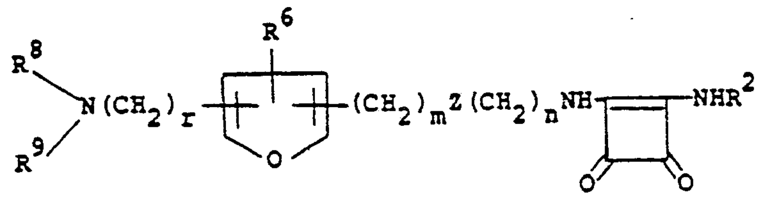

Ib

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

5. A process of Claim 1 wherein the compound of Formula I has the structure

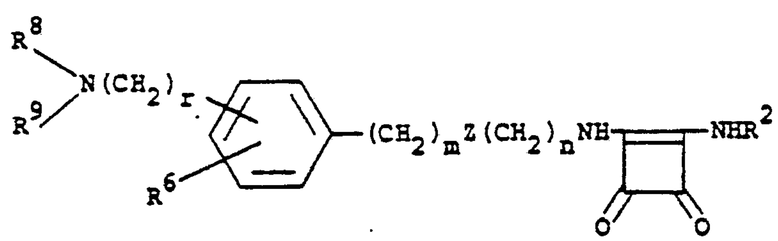

Ic

wherein

$R^2$ is hydrogen, $C_1$—$C_6$-alkyl, allyl, propargyl, 3-pyridylmethyl or 6-methyl-3-pyridylmethyl;

m is an integer of from 0 to 2 inclusive;

n is an integer of from 2 to 5 inclusive;

Z is sulfur, oxygen or methylene;

$R^6$ is hydrogen or $C_1$—$C_6$-alkyl;

r is an integer of from 1 to 4 inclusive; and

$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino,

thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

6. A process of Claim 1 wherein the compound of Formula I has the structure

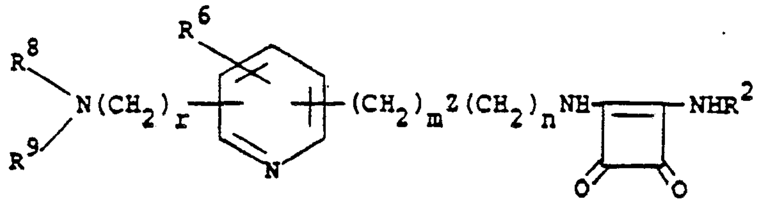

Id

wherein

$R^2$ is hydrogen, $C_1$—$C_6$-alkyl, allyl, propargyl, 3-pyridylmethyl or 6-methyl-3-pyridylmethyl;
m is an integer of from 0 to 2 inclusive;
n is an integer of from 2 to 5 inclusive;
Z is sulfur, oxygen or methylene;
$R^6$ is hydrogen or $C_1$—$C_6$-alkyl;
r is an integer of from 1 to 4 inclusive; and
$R^8$ and $R^9$ each are independently hydrogen or $C_1$—$C_6$-alkyl, or $R^8$ and $R^9$, taken together with the nitrogen atom to which they are attached, may be pyrrolidin-1-yl, methylpyrrolidin-1-yl, morpholino, thiomorpholino, piperidino, methylpiperidino, 4-methyl-1-piperazinyl, 1,2,3,6-tetrahydropyrid-1-yl, homopiperidino, heptamethyleneimino, octamethyleneimino or 3-azabicyclo[3.2.2]nonane, or a nontoxic, pharmaceutically acceptable acid addition salt, hydrate or solvate thereof; and a pharmaceutically acceptable carrier.

7. A process of Claim 5 wherein $R^2$ is hydrogen.

8. A process of any one of Claims 1—7 for preparing a composition, wherein the dosage of pepstatin is from 100 to 175 mg and the dosage of the compound of Formula I is from 2 to 100 mg.

9. A process of Claim 8 for preparing a composition, wherein the dosage of the compound of Formula I is from 4 to 50 mg.

10. A process of Claim 8 for preparing a composition which comprises from 100 to 175 mg of pepstatin and from 2 to 15 mg of 1-amino-2-[3-(3-piperidinomethylphenoxy)propylamino]cyclobutene-3,4-dione or a nontoxic, pharmaceutically acceptable acid addition salt, hydrate or solvate thereof; and a pharmaceutically acceptable carrier.

11. A process of Claim 8 for preparing a composition which comprises from 100 to 175 mg of pepstatin and from 2 to 15 mg of 1-amino-2-[3-(3-dimethylaminomethylphenoxy)propylamino]cyclobutene-3,4-dione, or a nontoxic, pharmaceutically acceptable acid addition salt, hydrate or solvate thereof; and a pharmaceutically acceptable carrier.

12. A process of Claims 8—11 for preparing a composition in which the pepstatin is in the form of pepstatin floating minicapsules.

13. A process of any one of Claims 1—12 for preparing a composition in unit dosage form.

**Patentansprüche für die Vertragsstaaten: BE CH DE FR GB IT LI LU NL SE**

1. Pharmazeutisches Mittel zur Behandlung von peptischem Ulcus, enthaltend eine die peptische Aktivität inhibierende Menge Pepstatin und eine antiulcerogen wirksame Menge wenigstens einer Verbindung der allgemeinen Formel I:

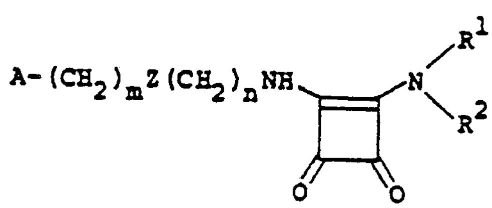

I

worin

$R^1$ und $R^2$ jeweils unabhängig voneinander für Wasserstoff oder $C_1$—$C_6$-Alkyl stehen, wobei, wenn $R^1$ Wasserstoff bedeutet, $R^2$ auch für Allyl, Propargyl, Cyclo-$C_3$—$C_7$-alkyl($C_1$—$C_6$)alkyl, Cyclo-$C_3$—$C_7$-alkyl, Cyano-$C_1$—$C_6$-alkyl, 2-Fluoroethyl, 2,2,2-Trifluoroethyl, Hydroxy, 2,3-Dihydroxypropyl,

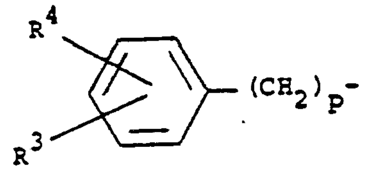
or
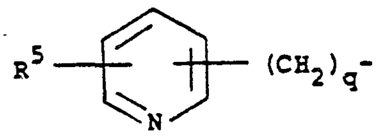

stehen kann,

p eine ganze Zahl von 1 bis 6 einschließlich bedeutet;

q eine ganze Zahl von 1 bis 6 einschließlich bedeutet,

$R^3$ und $R^4$ jeweils unabhängig voneinander für Wasserstoff, $C_1$—$C_6$-Alkyl, Hydroxy, $C_1$—$C_6$-Alkoxy oder Halogen stehen, wobei, wenn $R^3$ für Wasserstoff steht, $R^4$ auch Trifluormethyl bedeuten kann, oder $R^3$ und $R^4$ zusammen für Methylendioxy stehen können,

$R^5$ Wasserstoff bedeutet,

m eine ganze Zahl von 0 bis 2 einschließlich bedeutet;

n eine ganze Zahl von 2 bis 5 einschließlich bedeutet;

Z für Schwefel, Sauerstoff oder Methylen steht; und

A für

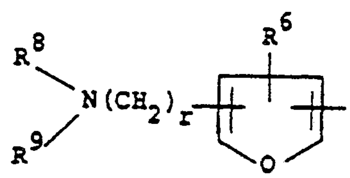
,
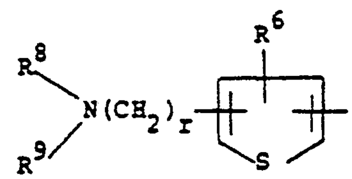
,

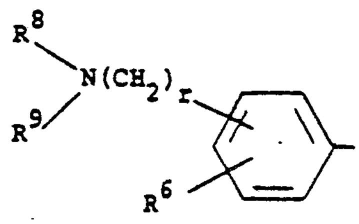
oder
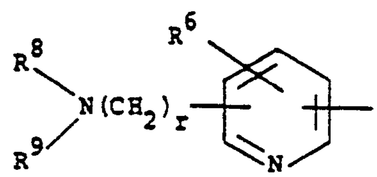

steht, wobei

$R^6$ für Wasserstoff, $C_1$—$C_6$-Alkyl, $C_1$—$C_6$-Alkoxy oder Halogen steht;

r eine ganze Zahl von 1 bis 4 einschließlich bedeutet; und

$R^8$ und $^9$ jeweils unabhängig voneinander für Wasserstoff, $C_1$—$C_6$-Alkyl, Allyl, Propargyl, $C_1$—$C_6$-Alkoxy-$C_1$—$C_6$-alkyl, in dem die $C_1$—$C_6$-Alkoxygruppe wenigstens zwei Kohlenstoffatome vom Stickstoffatom entfernt ist, Cyclo-$C_3$—$C_7$-alkyl oder Phenyl-$C_1$—$C_6$-alkyl stehen, wobei $R^8$ und $R^9$ nicht beide für Cyclo-$C_3$—$C_7$-alkyl stehen können, oder $R^8$ und $R^9$ zusammen mit dem Stickstoffatom, an das sie gebunden sind, Pyrrolidin-1-yl, Methylpyrrolidin-1-yl, Dimethylpyrrolidin-1-yl, Morpholino, Thiomorpholino, Piperidino, Methylpiperidino, Dimethylpiperidino, Hydroxypiperidino, 4-Methyl-1-piperazinyl, 1,2,3,6-Tetrahydropyrid-1-yl, 3-Pyrrolin-1-yl, Homopiperidino, Heptamethylenimino, Octamethylenimino oder 3-Azabicyclo[3.2.2]nonan bedeuten, oder ein nichttoxisches, pharmazeutisch verträgliches Säureadditionssalz, Hydrat oder Solvat davon; und einen pharmazeutisch vertraglichen Träger.

2. Mittel nach Anspruch 1, wobei

$R^1$ und $R^2$ jeweils unabhängig voneinander für Wasserstoff oder $C_1$—$C_6$-Alkyl stehen, wobei, wenn $R^1$ Wasserstoff bedeutet, $R^2$ auch Allyl, Propargyl, $C_3$—$C_7$-Cycloalkyl-$C_1$—$C_6$-alkyl,

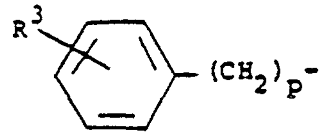
oder
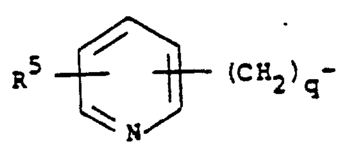

worin

p und q jeweils unabhängig voneinander eine ganze Zahl von 1 bis 6 einschließlich bedeuten und $R^2$ und $R^5$ für Wasserstoff, $C_1$—$C_6$-Alkyl oder $C_1$—$C_6$-Alkoxy stehen, bedeuten können;

m eine ganze Zahl von 0 bis 2 einschließlich bedeutet;

n eine ganze Zahl von 2 bis 5 einschließlich bedeutet;

Z für Schwefel, Sauerstoff oder Methylen steht; und

A für

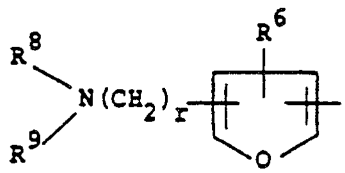 , 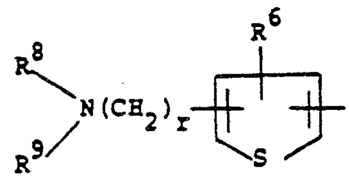 ,

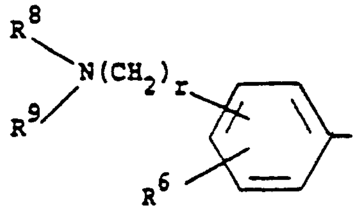 oder 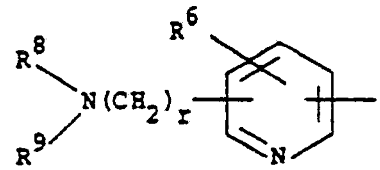

steht, worin

$R^6$ für Wasserstoff, $C_1$—$C_6$-Alkyl oder $C_1$—$C_6$-Alkoxy steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeutet oder

$R^8$ und $R^9$ zusammen mit den Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2.]-nonan steht, oder ein nicht-toxisches, pharmazeutisch verträgliches Säureadditionssalz, Hydrat oder Solvat davon; und einen pharmazeutisch verträglichen Träger.

3. Mittel nach Anspruch 1, wobei die Verbindung der Formel I die Struktur

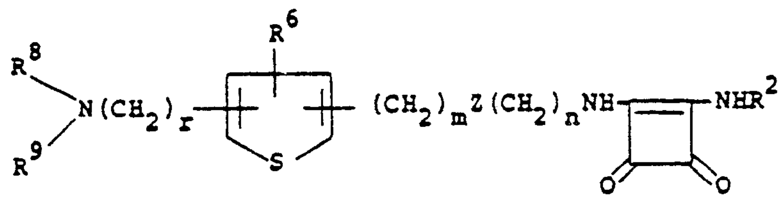 Ia

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

4. Mittel nach Anspruch 1, wobei die Verbindung der Formel I die Struktur

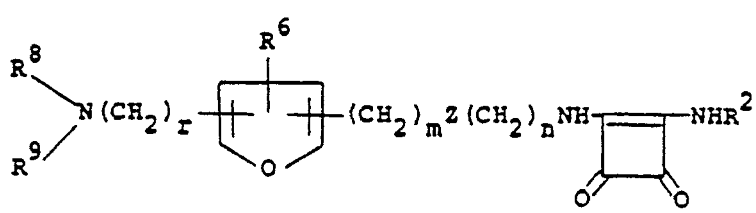 Ib

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

5. Mittel nach Anspruch 1, wobei die Verbindung der Formel I die Struktur

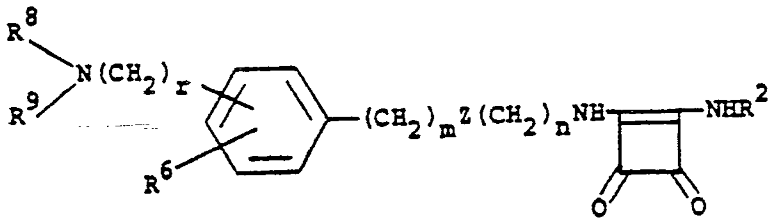

Ic

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.3.2]-nonan, oder ein nichttoxisches, pharmazeutisch verträgliches Säureadditionssalz, Hydrat oder Solvat davon; und einem pharmazeutisch verträglichen Träger.

6. Mittel nach Anspruch 1, wobei die Verbindung der Formel I die Struktur

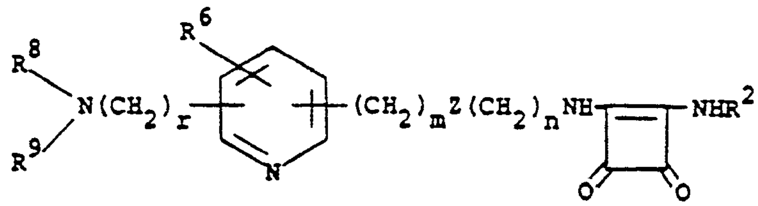

Id

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

7. Mittel nach Anspruch 5, wobei $R^2$ für Wasserstoff steht.

8. Mittel nach einem der Ansprüche 1—7, wobei die Menge an Pepstatin 100 bis 175 mg beträgt und die Menge an Verbindung der Formel I 2 bis 100 mg beträgt.

9. Mittel nach Anspruch 8, wobei die Menge an Verbindung der Formel I 4 bis 50 mg beträgt.

10. Pharmazeutisches Mittel nach Anspruch 8, enthaltend 100 bis 175 mg Pepstatin und 2 bis 15 mg 1-Amino-2-[3-(3-piperidinomethylphenoxy)propylamino]-cyclobuten-3,4-dion oder ein nicht-toxisches, pharmazeutisch verträgliches Säureadditionssalz, Hydrat oder Solvat davon, und einen pharmazeutisch verträglichen Träger.

11. Pharmazeutisches Mittel nach Anspruch 8, enthaltend 100 bis 175 mg Pepstatin und 2 bis 15 mg 1-Amino-2-[3-(3-dimethylaminomethylphenoxy)-propylamino]cyclo-buten-3,4-dion, oder ein nicht-toxisches, pharmazeutisch verträgliches Säureadditionssalz, Hydrat oder Solvat davon, und einen pharmazeutisch verträlichen Träger.

12. Mittel nach den Ansprüchen 8 bis 11, wobei das Pepstatin in Form von floatenden Pepstatin-Minikapseln vorliegt.

13. Mittel nach einem der Ansprüche 1—12 in Einheitsdosisform.

**Patentansprüche für den Vertragsstaat: AT**

1. Verfahren zur Herstellung eines pharmazeutischen Mittels zur Behaldlung von peptischem Ulcus, wobei man eine die peptische Aktivität-inhibierende Menge Pepstatin und eine anti-ulcerogen wirksame Menge wenigstens einer Verbindung der Formel I:

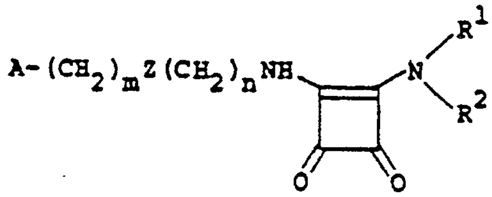

I

worin

$R^1$ und $R^2$ jeweils unabhängig voneinander für Wasserstoff oder $C_1$—$C_6$-Alkyl stehen, wobei, wenn $R^1$ Wasserstoff bedeutet, $R^2$ auch für Allyl, Propargyl, Cyclo-$C_3$—$C^7$-alkyl($C_1$—$C_6$)alkyl, Cyclo-$C_3$—$C_7$-alkyl, Cyano-$C_1$—$C_6$-alkyl, 2-Fluoroethyl, 2,2,2-Trifluoroethyl, Hydroxy, 2,3-Dihydroxypropyl,

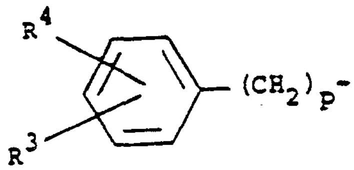

oder

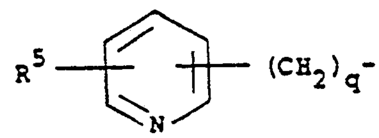

worin

p eine ganze Zahl von 1 bis 6 einschließlich bedeutet;

q eine ganze Zahl von 1 bis 6 einschließlich bedeutet, stehen kann,

$R^3$ und $R^4$ jeweils unabhängig voneinander für Wasserstoff, $C_1$—$C_6$-Alkyl, Hydroxy, $C_1$—$C_6$-Alkoxy oder Halogen stehen, wobei, wenn $R^3$ für Wasserstoff steht, $R^4$ auch Trifluormethyl bedeuten kann oder $R^3$ und $R^4$ zusammen für Methylendioxy stehen können,

$R^5$ Wasserstoff bedeutet,

m eine ganze Zahl von 0 bis 2 einschließlich bedeutet;

n eine ganze Zahl von 2 bis 5 einschließlich bedeutet;

Z für Schwefel, Sauerstoff oder Methylen steht; und

A für

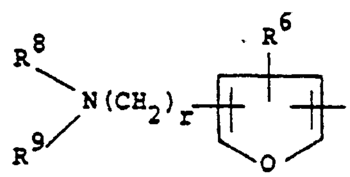

,

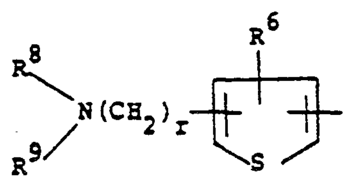

,

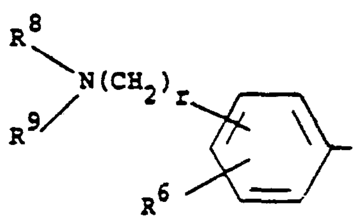

oder

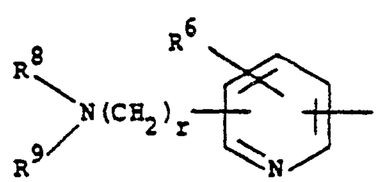

steht, wobei

$R^6$ für Wasserstoff, $C_1$—$C_6$-Alkyl, $C_1$—$C_6$-Alkoxy oder Halogen steht;

r eine ganze Zahl von 1 bis 4 einschließlich bedeutet; und

$R^8$ und $R^9$ jeweils unabhängig voneinander für Wasserstoff, $C_1$—$C_6$-Alkyl, Allyl, Propargyl, $C_1$—$C_6$-Alkoxy-$C_1$—$C_6$-alkyl, in dem die $C_1$—$C_6$-Alkoxygruppe wenigstens zwei Kohlenstoffatome vom Stickstoffatom entfernt ist, Cyclo-$C_3$—$C_7$-alkyl oder Phenyl-$C_1$—$C_6$-alkyl stehen, wobei $R^8$ und $R^9$ nicht beide für Cyclo-$C_3$—$C_7$-alkyl stehen können, oder $R^8$ und $R^9$ zusammen mit dem Stickstoffatom, an das sie gebunden

sind, Pyrrolidin-1-yl, Methylpyrrolidin-1-yl. Dimethylpyrrolidin-1-yl, Morpholino, Thiomorpholino, Piperidino, Methylpiperidino, Dimethylpiperidino, Hydroxypiperidino, 4-Methyl-1-piperazinyl, 1,2,3,6-Tetrahydropyrid-1-yl, 3-Pyrrolin-1-yl, Homopiperidino, Heptamethylenimino, Octamethylenimino oder 3-Azabicyclo[3.2.2]nonan bedeuten, oder ein nichttoxisches, pharmazeutisch verträgliches Säureadditionssalz, Hydrat oder Solvat davon; und einen pharmazeutisch verträglichen Träger vermischt.

2. Verfahren nach Anspruch 1, wobei $R^1$ und $R^2$ jeweils unabhängig voneinander für Wasserstoff oder $C_1$—$C_6$-Alkyl stehen, wobei, wenn $R^1$ Wasserstoff bedeutet, $R^2$ auch Allyl, Propargyl, $C_3$—$C_7$-Cycloalkyl-$C_1$—$C_6$-alkyl,

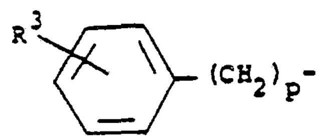

oder

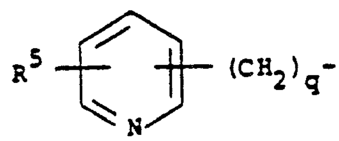

worin

p und q jeweils unabhängig voneinander eine ganze Zahl von 1 bis 6 einschließlich bedeuten und $R^2$ und $R^5$ für Wasserstoff, $C_1$—$C_6$-Alkyl oder $C_1$—$C_6$-Alkoxy stehen, bedeuten können;

m eine ganze Zahl von 0 bis 2 einschließlich bedeutet;

n eine ganze Zahl von 2 bis 5 einschließlich bedeutet;

Z für Schwefel, Sauerstoff oder Methylen steht; und

A für

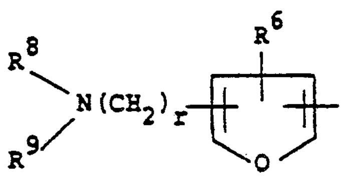

,

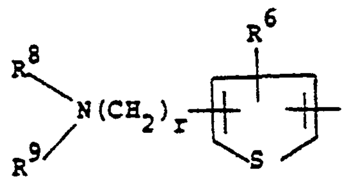

,

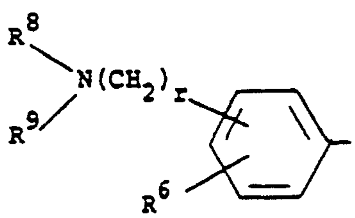

oder

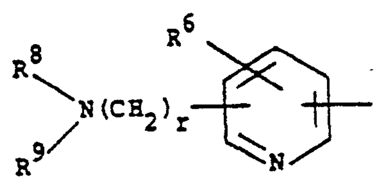

steht, worin

$R^6$ für Wasserstoff, $C_1$—$C_6$-Alkyl oder $C_1$—$C_6$-Alkoxy steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeutet oder

$R^8$ und $R^9$ zusammen mit den Stickstoffatomen, an das sie gebunden sind für pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethyleninino oder 3-Azabicyclo[3.3.2.]-nonan steht, oder ein nicht-toxisches, pharmazeutisch verträliches Säureadditionssalz, Hydrat oder Solvat davon; und einen pharmazeutisch verträglichen Träger.

3. Verfahren nach Anspruch 1, wobei die Verbindung der Formel I die Struktur

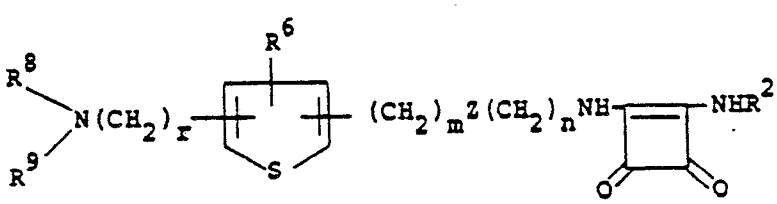

Ia

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

4. Verfahren nach Anspruch 1, wobei die Verbindung der Formel I die Struktur

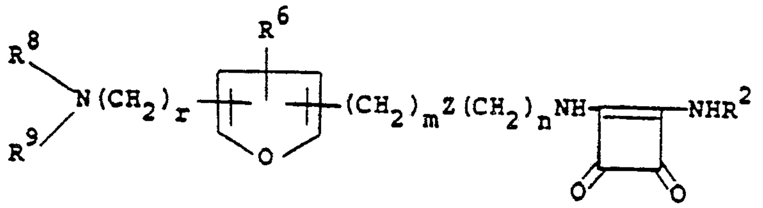

Ib

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

5. Verfahren nach Anspruch 1, wobei die Verbindung der Formel I die Struktur

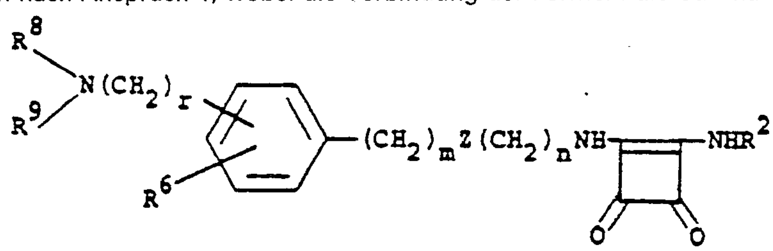

Ic

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_1$—$C_6$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

6. Verfahren nach Anspruch 1, wobei die Verbindung der Formel I die Struktur

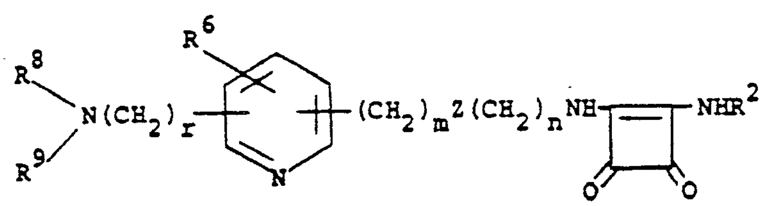

Id

besitzt, worin $R^2$ für Wasserstoff, $C_1$—$C_6$-Alkyl, Allyl, Propargyl, 3-Pyridylmethyl oder 6-Methyl-3-pyridylmethyl steht;

m eine ganze Zahl von 0 bis 2 einschließlich bedeutet;

n eine ganze Zahl von 2 bis 5 einschließlich bedeutet;

Z für Schwefel, Sauerstoff oder Methylen steht;

$R^6$ für Wasserstoff oder $C_{1-6}$-Alkyl steht;

r eine ganze Zahl von 1 bis 4 einschließlich bedeutet; und

$R^8$ und $R^9$ jeweils unabhängig voneinander Wasserstoff oder $C_1$—$C_6$-Alkyl bedeuten oder $R^8$ und $R^9$ zusammen mit dem Stickstoffatom, an das sie gebunden sind für Pyrrolidin-1-yl, Methylpyrrolidin-1-yl, Morpholino, Thiomorpholino, Piperidino, Methylpiperidino, 4-Methyl-1-piperazinyl, 1,2,3,6-Tetrahydropyrid-1-yl, Homopiperidino, Heptamethylenimino, Octamethylenimino oder 3-Azabicyclo[3.2.2]-nonan, oder ein nichttoxisches, pharmazeutisch verträgliches Säureadditionssalz, Hydrat oder Solvat davon; und einem pharmazeutisch verträglichen Träger.

7. Verfahren nach Anspruch 5, wobei $R^2$ für Wasserstoff steht.

8. Verfahren nach einem der Ansprüche 1—7 zur Herstellung eines Mittels, bei dem die Menge an Pepstatin 100 bis 175 mg beträgt und die Menge an Verbindung der Formel I 2 bis 100 mg beträgt.

9. Verfahren nach Anspruch 8 zur Herstellung eines Mittels, bei dem die Menge an Verbindung der Formel I 4 bis 50 mg beträgt.

10. Verfahren nach Anspruch 8 zur Herstellung eines Mittels, das 100 bis 175 mg pepstatin und 2 bis 15 mg 1-Amino-2-[3-(3-piperidinomethylphenoxy)propylamino]-cyclobuten-3,4-dion oder ein nicht-toxisches, pharmazeutisch verträgliches Säureadditionssalz, Hydrat oder Solvat davon, und einen pharmazeutisch verträglichen Träger enthält.

11. Verfahren nach Anspruch 8 zur Herstellung eines Mittels, das 100 bis 175 mg Pepstatin und 2 bis 15 mg 1-Amino-2-[3-(3-dimethylaminomethylphenoxy)-propylamino]cyclobuten-3,4-dion, oder ein nicht-toxisches, pharmazeutisch verträgliches Säureadditionssalz, Hydrat oder Solvat davon, und einen pharmazeutisch verträglichen Träger enthält.

12. Verfahren nach den Ansprüchen 8 bis 11 zur Herstellung eines Mittels, in dem das Pepstatin in Form von floatenden Pepstatin-Minikapseln vorliegt.

13. Verfahren nach einem der Ansprüche 1—12 zur Herstellung eines Mittels in Einheitsdosisform.

**Revendications pour les Etats contractants: BE CH DE FR GB IT LI LU NL SE**

1. Un composition pharmaceutique utile pour les traitements d'ulcères peptiques qui comprend une quantité inhibitrice de l'activité peptique de pepstatine et une quantité anti-ulcérogène efficace d'au moins un composé de formule (I):

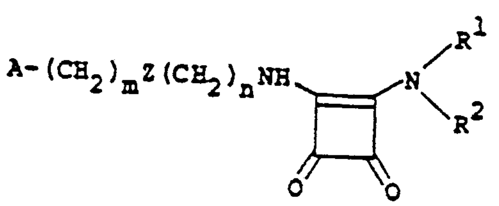

I

dans laquelle:

$R^1$ et $R^2$ indépendamment l'un de l'autre, sont un atome d'hydrogène ou un radical $C_1$—$C_6$-alkyle et lorsque $R^1$ est un atome d'hydrogène $R^2$ peut être un radical allyle, propargyle, cyclo-$C_3$—$C_7$-alkyle-$C_1$—$C_6$-alkyle, cyclo-$C_3$—$C_7$-alkyle, cyano-$C_1$—$C_6$-alkyle, 2-fluoroéthyle, 2,2,2-trifluoroéthyle, hydroxy, 2,3-dihydroxypropyle:

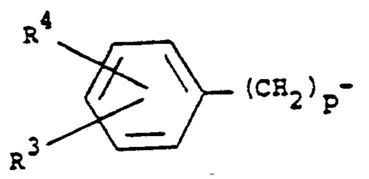

or

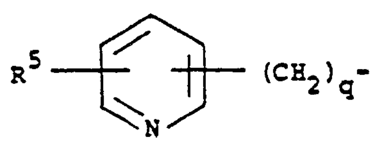

dans lesquelles:

p est un nombre entier compris entre 1 et 6 bornes incluses;

q est un nombre entier compris entre 1 et 6 bornes incluses;

$R^3$ et $R^4$, indépendamment l'un de l'autre, sont un atome d'hydrogène ou un radical $C_1$—$C_6$-alkyle, hydroxy, $C_1$—$C_6$-alkoxy ou un halogène, et lorsque $R^3$ est un atome d'hyrogène, $R^4$ peut également être un radical trifluorométhyle, ou encore $R^3$ et $R^4$ forment ensemble un radical méthylènedioxy;

$R^5$ est un atome d'hyrogène;

m est un nombre entier compris entre 0 et 2 bornes incluses;

n est un nombre entier compris entre 2 et 5 bornes incluses;

Z est un atome de soufre, d'oxygène ou un groupe méthylène; et

A est

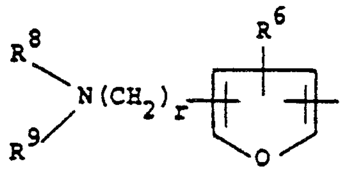 , 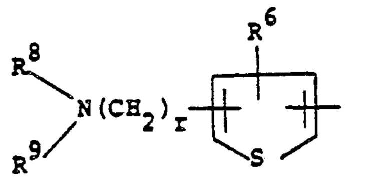 ,

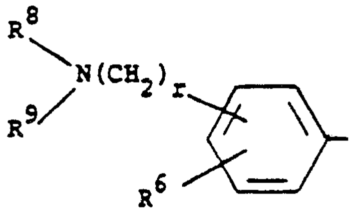 ou 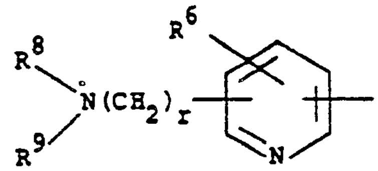

dans lesquels:

$R^6$ est un atome d'hydrogène, un radical $C_1$—$C_6$-alkyle, $C_1$—$C_6$-alkoxy ou un halogène;

r est un nombre entier compris entre 1 et 4 bornes incluses; et

$R^8$ et $R^9$, indépendamment l'un de l'autre, sont un atome d'hydrogène, un radical $C_1$—$C_6$-alkyle, allyle, propargyle, $C_1$—$C_6$-alkoxy-$C_1$—$C_6$-alkyle dans lequel la partie $C_1$—$C_6$-alkoxy comprend au moins deux atomes de carbone séparés de l'atome d'azote, cyclo-$C_3$—$C_7$-alkyle, ou phényl-$C_1$—$C_6$-alkyle, étant entendu que $R^8$ et $R^9$ peuvent ne pas être simultanément un radical cyclo-$C_3$—$C_7$-alkyle, ou encore que $R^8$ et $R^9$ pris ensemble avec l'atome d'azote auquel ils sont liés peuvent être un groupe pyrrolidine-1-yle, méthylpyrrolidine-1-yle, diméthylpyrrolidine-1-yle, morpholino, thiomorpholino, pipéridino, méthylpipéridino, diméthylpipéridino, hydroxypipéridino, 4-méthyl-1-pipérazinyle, 1,2,3,6-tétrahydropyride-1-yle, 3-pyrroline-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

2. Un composé selon la revendication 1, dans lequel $R^1$ et $R^2$, indépendamment l'un de l'aùtre, sont un atome d'hydrogène ou un radical $C_1$—$C_6$-alkyle, et lorsque $R^1$ est un atome d'hydrogène, $R^2$ peut également être un radical allyle, propargyle, $C_3$—$C_7$-cyclo-alkyle-$C_1$—$C_6$-alkyle,

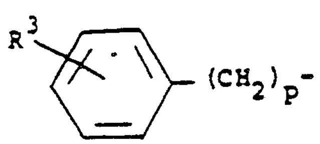 ou 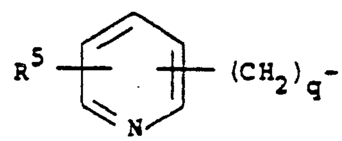

dans lesquels:

p et q sont, indépendamment l'un de l'autre, un nombre entier compris entre 1 et 6 bornes incluses; et

$R^3$ et $R^5$ sont, indépendamment l'un de l'autre, un atome d'hydrogène, un radical $C_1$—$C_6$-alkyle ou $C_1$-alkoxy;

m est un nombre entier compris entre 0 et 2 bornes incluses;

n est un nombre entier compris entre 2 et 5 bornes incluses;

Z est un atome de soufre ou d'oxygène ou un groupe méthylène; et

A est

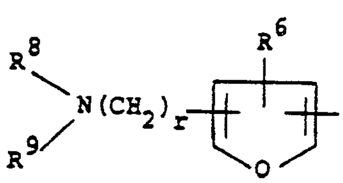 , 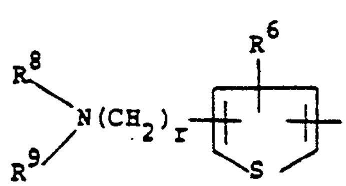 ,

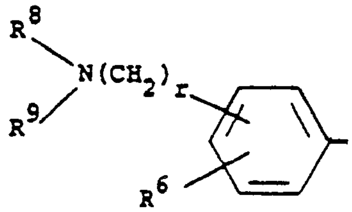 ou 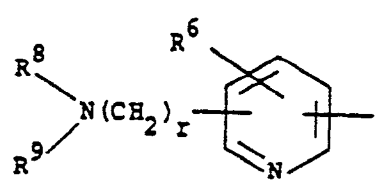

dans lesquels:

$R^6$ est un atome d'hydrogène, un radical $C_1$—$C_6$-alkyle ou $C_1$—$C_6$-alkoxy;

r est un nombre entier compris entre 1 et 4 bornes incluses; et

$R^8$ et $R^9$ sont indépendamment l'un de l'autre, un atome d'hydrogène ou un radical $C_1$—$C_6$-alkyle ou encore $R^8$ et $R^9$ pris ensemble avec l'atome d'azote auquel ils sont liés peuvent être un groupe pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

3. Une composition selon la revendication 1, dans laquelle le composé de formule (I) présente la structure:

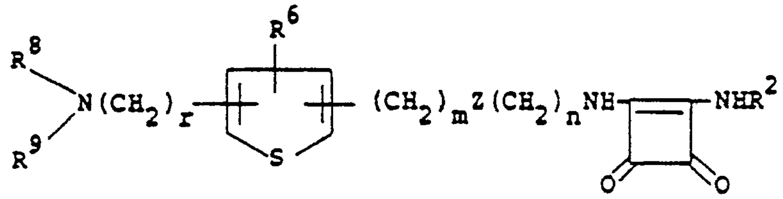

Ia

dans laquelle:

$R^2$ est un atome d'hydrogène, un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

4. Une composition selon la revendication 1, dans laquelle le composé de formule (I) présente la structure:

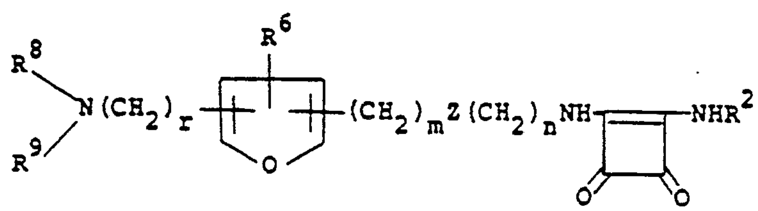

Ib

dans laquelle:

$R^2$ est un atome d'hydrogène, un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

5. Une composition selon la revendication 1, dans laquelle le composé de formule (I) présente la structure:

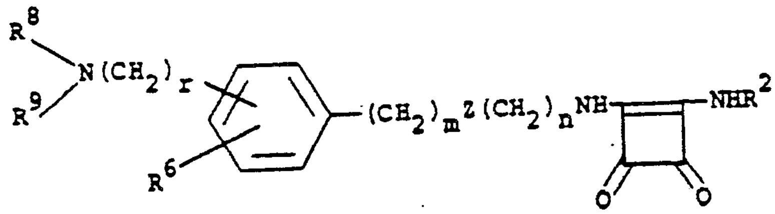

Ic

dans laquelle:

$R^2$ est un atome d'hydrogène ou un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

6. Une composition selon la revendication 1, dans laquelle le composé de formule (I) présente la structure:

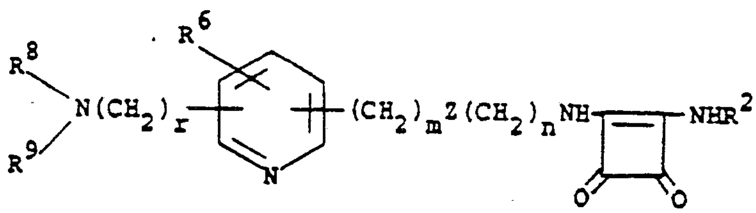

Id

dans laquelle:

$R^2$ est un atome d'hydrogène ou un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

7. Une composition selon la revendication 5, dans laquelle $R^2$ est l'hydrogène.

8. Une composition selon l'une quelconque des revendications 1 à 7, dans laquelle la dose de pepstatine est de 100 à 175 mg et la dose du composé de formule (I) est de 2 à 100 mg.

9. Une composition selon la revendication 8, dans laquelle la dose du composé de formule (I) est de 4 à 50 mg.

10. Une composition pharmaceutique selon la revendication 8, qui comprend de 100 à 175 mg de pepstatine et de 2 à 15 mg de 1-amino-2-{3-(3-pipéridinométhylphénoxy)propylamino}-cyclobutène-3,4-dione ou un sel d'addition d'acide, hydrate ou solvate, pharmaceutiquement acceptable et non toxique en dérivant;

ainsi qu'un support pharmaceutiquement acceptable.

11. Une composition selon la revendication 8, qui comprend de 100 à 175 mg de pepstatine et de 2 à 15 mg de 1-amino-2-{3-(3-diméthylaminométhylphénoxy)-propylamino}-cyclobutène-3,4-dione ou un sel

d'addition d'acide, hydrate ou solvate, pharmaceutiquement acceptable et non toxique en dérivant; ainsi qu'un support pharmaceutiquement acceptable.

12. Une composition selon l'une quelconque des revendications 8 à 11, dans laquelle la pepstatine est sous forme de mini capsules flottantes de pepstatine.

13. Une composition selon l'une quelconque des revendications 8 à 12, sous forme de dose unitaire.

**Revendications pour l'Etat contractant: AT**

1. Un procédé de préparation d'une composition pharmaceutique utile pour les traitements d'ulcères peptiques qui consiste à mélanger une quantité inhibitrice d'activité peptique de pepstatine et une quantité anti-ulcérogène efficace d'au moins un composé de formule (I):

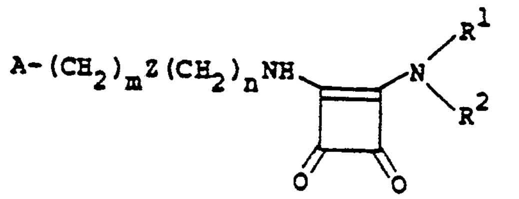

I

dans laquelle:

$R^1$ et $R^2$ indépendamment l'un de l'autre, sont un atome d'hydrogène ou un radical $C_1$—$C_6$-alkyle et lorsque $R^1$ est un atome d'hydrogène $R^2$ peut être un radical allyle, propargyle, cyclo-$C_3$—$C_6$-alkyle-$C_1$—$C_6$-alkyle, cyclo-$C_3$—$C_7$-alkyle, cyano-$C_1$—$C_6$-alkyle, 2-fluoroéthyle, 2,2,2-trifluoroéthyle, hydroxy, 2,3-dihydroxypropyle:

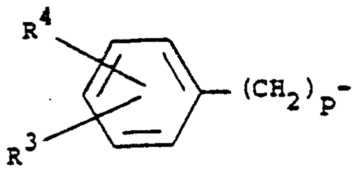

ou

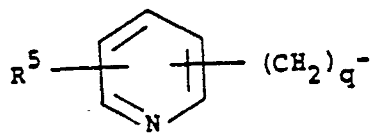

dans lesquelles:

p est un nombre entier compris entre 1 et 6 bornes incluses;

q est un nombre entier compris entre 1 et 6 bornes incluses;

$R^3$ et $R^4$, indépendamment l'un de l'autre, sont un atome d'hydrogène ou un radical $C_1$—$C_6$-alkyle, hydroxy, $C_1$—$C_6$-alkoxy ou un halogène, et lorsque $R^3$ est un atome d'hyrogène, $R^4$ peut également être un radical trifluorométhyle, ou encore $R^3$ et $R^4$ forment ensemble un radical méthylènedioxy;

$R^5$ est un atome d'hyrogène;

m est un nombre entier compris entre 0 et 2 bornes incluses;

n est un nombre entier compris entre 2 et 5 bornes incluses;

Z est un atome de soufre, d'oxygène ou un groupe méthylène; et

A est

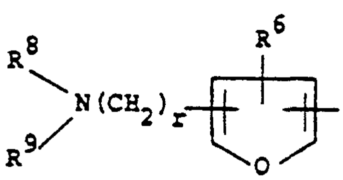

,

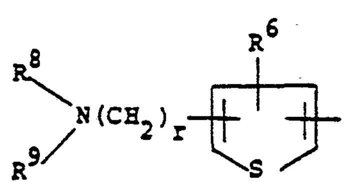

,

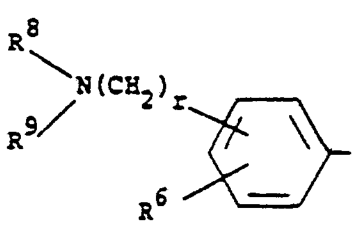

ou

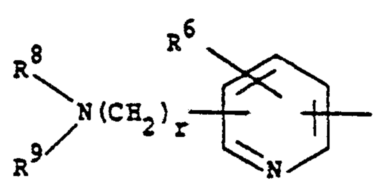

dans lesquels:

$R^6$ est un atome d'hydrogène, un radical $C_1$—$C_6$-alkyle, $C_1$—$C_6$-alkoxy ou un halogène;

r est un nombre entier compris entre 1 et 4 bornes incluses; et

$R^8$ et $R^9$, indépendamment l'un de l'autre, sont un atome d'hydrogène, un radical $C_1$—$C_6$-alkyle, allyle, propargyle, $C_1$—$C_6$-alkoxy-$C_1$—$C_6$-alkyle dans lequel la partie $C_1$—$C_6$-alkoxy comprend au moins deux atomes de carbone séparés de l'atome d'azote, cyclo-$C_3$—$C_7$-alkyle, ou phényl-$C_1$—$C_6$-alkyle, étant entendu que $R^8$ et $R^9$ peuvent ne pas être simultanément un radical cyclo-$C_3$—$C_7$-alkyle, ou encore que $R^8$ et $R^9$ pris ensemble avec l'atome d'azote auquel ils sont liés peuvent être un groupe pyrrolidine-1-yle, méthylpyrrolidine-1-yle, diméthylpyrrolidine-1-yle, morpholino, thiomorpholino, pipéridino, méthylpipéridino, diméthylpipéridino, hydroxypipéridino, 4-méthyl-1-pipérazinyle, 1,2,3,6-tétrahydropyride-1-yle, 3-pyrroline-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

2. Un procédé selon la revendication 1, dans lequel $R^1$ et $R^2$, indépendamment l'un de l'autre, sont un atome d'hydrogène ou un radical $C_1$—$C_6$-alkyle, et lorsque $R^1$ est un atome d'hydrogène, $R^2$ peut également être un radical allyle, propargyle, $C_3$—$C_7$-cyclo-alkyle-$C_1$—$C_6$-alkyle,

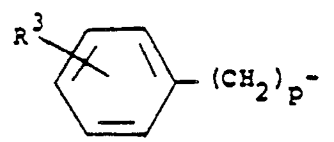

ou

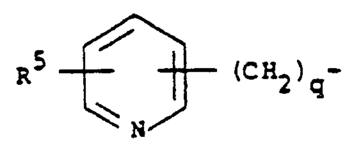

dans lesquels:

p et q sont, indépendamment l'un de l'autre, un nombre entier compris entre 1 et 6 bornes incluses; et

$R^3$ et $R^5$ sont, indépendamment l'un de l'autre, un atome d'hydrogène, un radical $C_1$—$C_6$-alkyle ou $C_1$-alkoxy;

m est un nombre entier compris entre 0 et 2 bornes incluses;

n est un nombre entier compris entre 2 et 5 bornes incluses;

Z est un atome de soufre ou d'oxygène ou un groupe méthylène; et

A est

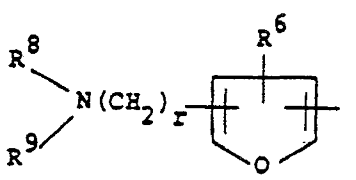

,

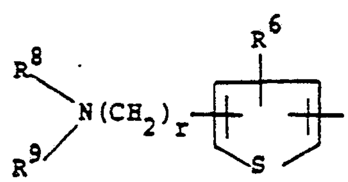

,

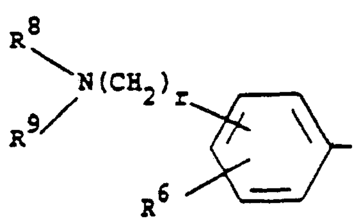

ou

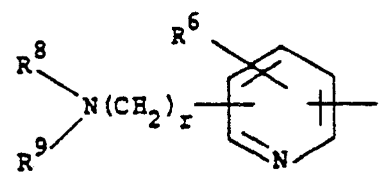

.

dans lesquels:

$R^6$ est un atome d'hydrogène, un radical $C_1$—$C_6$-alkyle ou $C_1$—$C_6$-alkoxy;

r est un nombre entier compris entre 1 et 4 bornes incluses; et

$R^8$ et $R^9$ sont indépendamment l'un de l'autre, un atome d'hydrogène ou un radical $C_1$—$C_6$-alkyle ou encore $R^8$ et $R^9$ pris ensemble avec l'atome d'azote auquel ils sont liés peuvent être un groupe pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

3. Un procéde selon la revendication 1, dans lequel le composé de formule (I) présente la structure:

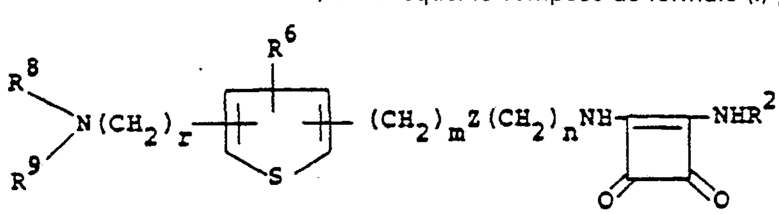

Ia

dans laquelle:

$R^2$ est un atome d'hydrogène, un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

4. Un procédé selon la revendication 1, dans lequel le composé de formule (I) présente la structure:

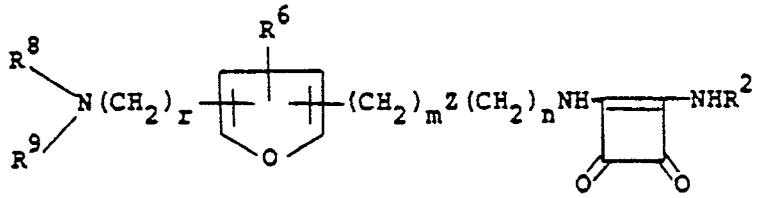

Ib

dans laquelle:

$R^2$ est un atome d'hydrogène, un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

5. Un procédé selon la revendication 1, dans lequel le composé de formule (I) présente la structure:

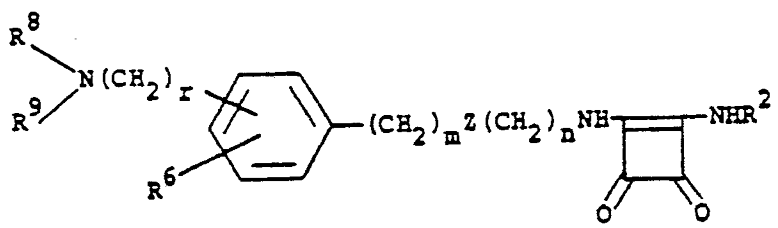

Ic

dans laquelle:

$R^2$ est un atome d'hydrogène ou un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

6. Un procédé selon la revendication 1, dans lequel le composé de formule (I) présente la structure:

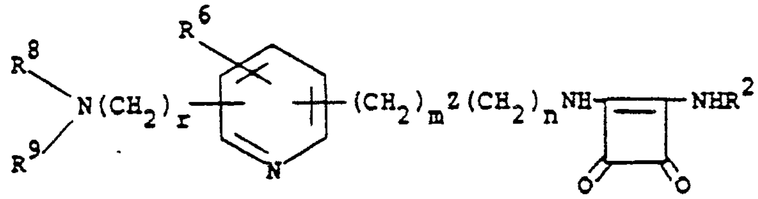

Id

dans laquelle:

$R^2$ est un atome d'hydrogène ou un radical $C_1$—$C_6$-alkyle, allyle, propargyle, 3-pyridylméthyle ou 6-méthyl-3-pyridylméthyle;

m est un nombre entier compris entre 0 et 2 bornes incluses;

n est un nombre entier compris entre 2 et 5 bornes incluses;

Z est un atome de soufre, d'oxygène ou un groupe méthylène;

$R^6$ est un atome d'hydrogène ou un radical $C_1$—$C_6$-alkyle;

r est un nombre entier compris entre 1 et 4 bornes incluses; et

$R^8$ et $R^9$ sont, indépendamment l'un de l'autre, un atome d'hydrogène ou un radical $C_1$—$C_6$-alkyle, ou $R^8$ et $R^9$, pris ensemble avec l'atome d'azote auquel ils sont liés peuvent être un radical pyrrolidine-1-yle, méthylpyrrolidine-1-yle, morpholino, thiomorpholino, pipéridino, méthylpipéridino, 4-méthyl-1-pipérazinyle, 1,2,3,6-tétrahydropyride-1-yle, homopipéridino, heptaméthylèneimino, octaméthylèneimino ou 3-azabicyclo(3,2,2)nonane;

ou un sel d'addition d'acide, hydrate ou solvate, pharmaceutiquement acceptable et non toxique en dérivant;

ainsi qu'un support pharmaceutiquement acceptable.

7. Un procédé selon la revendicaiton 5, dans lequel $R^2$ est l'hydrogène.

8. Un procédé selon l'une quelconque des revendications 1 à 7, dans lequel la dose de pepstatine est de 100 à 175 mg et la dose du composé de formule (I) est de 2 à 100 mg.

9. Un procédé selon la revendication 8, dans lequel la dose du composé de formule (I) est de 4 à 50 mg.

10. Un procédé pharmaceutique selon la revendication 8, qui comprend de 100 à 175 mg de pepstatine et de 2 à 15 mg de 1-amino-2-{3-(3-pipéridinométhylphénoxy)propylamino}-cyclobutène-3,4-dione ou un sel d'addition d'acide, hydrate ou solvate, pharmaceutiquement acceptable et non toxique en dérivant; ainsi qu'un support pharmaceutiquement acceptable.

11. Un procédé selon la revendication 8, qui comprend de 100 à 175 mg de pepstatine et de 2 à 15 mg de 1-amino-2-{3-(3-diméthylaminométhylphénoxy)-propylamino}-cyclobutène-3,4-dione ou un sel d'addition d'acide, hydrate ou solvate, pharmaceutiquement acceptable et non toxique en dérivant; ainsi qu'un support pharmaceutiquement acceptable.

12. Un procédé selon l'une quelconque des revendications 8 à 11, dans lequel la pepstatine est sous forme de mini capsules flottantes de pepstatine.

13. Un procédé selon l'une quelconque des revendications 8 à 12, pour préparer une composition sous forme de dose unitaire.

FIG. I

COMPARATIVE EFFECTS OF THE COMPOUND OF EXAMPLE 1 AND COMBINATIONS OF THE COMPOUND OF EXAMPLE 1 WITH PEPSTATIN IN THE PREVENTION OF HCl INDUCED GASTRIC EROSIONS IN THE RAT

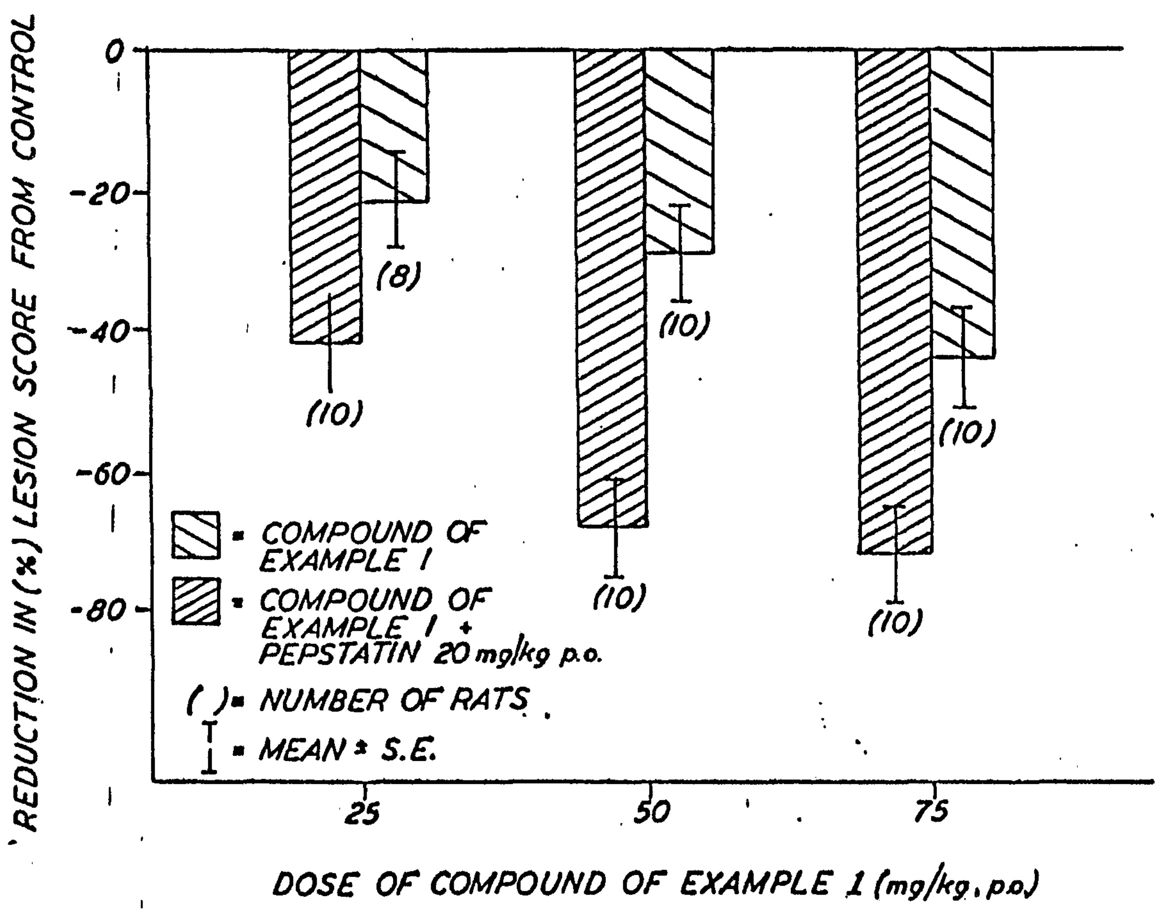

FIG. 2

DOSE RELATED EFFECTS OF THE COMPOUND OF EXAMPLE 1 AND COMBINATIONS OF THE COMPOUND OF EXAMPLE 1 WITH PEPSTATIN (20 mg./kg.) ON THE REDUCTION OF HCl INDUCED GASTRIC EROSIONS IN THE RAT

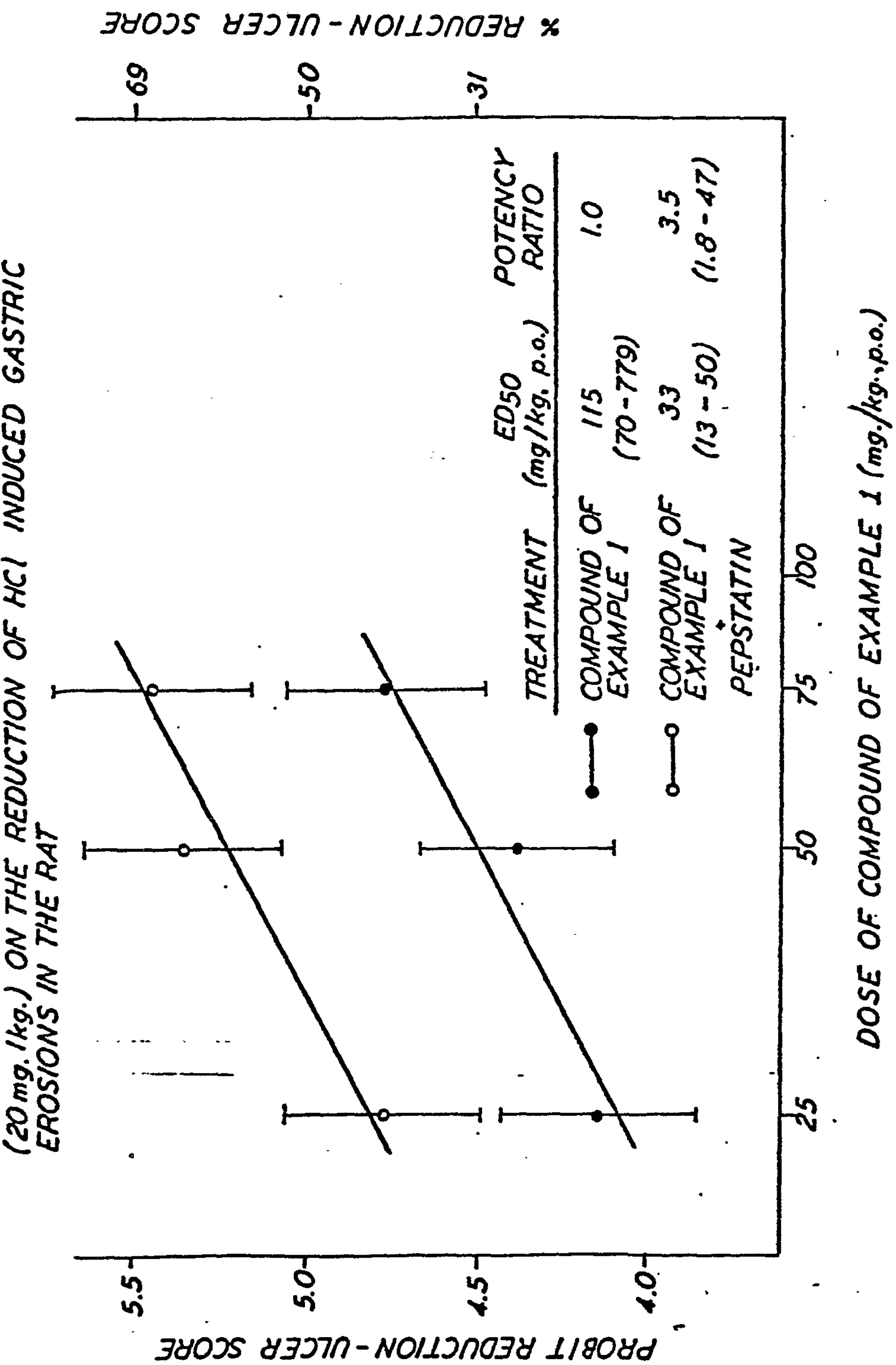

FIG. 3

COMPARATIVE EFFECTS OF RANITIDINE AND COMBINATIONS OF RANITIDINE WITH PEPSTATIN IN THE PREVENTION OF HCl INDUCED GASTRIC EROSIONS IN THE RAT

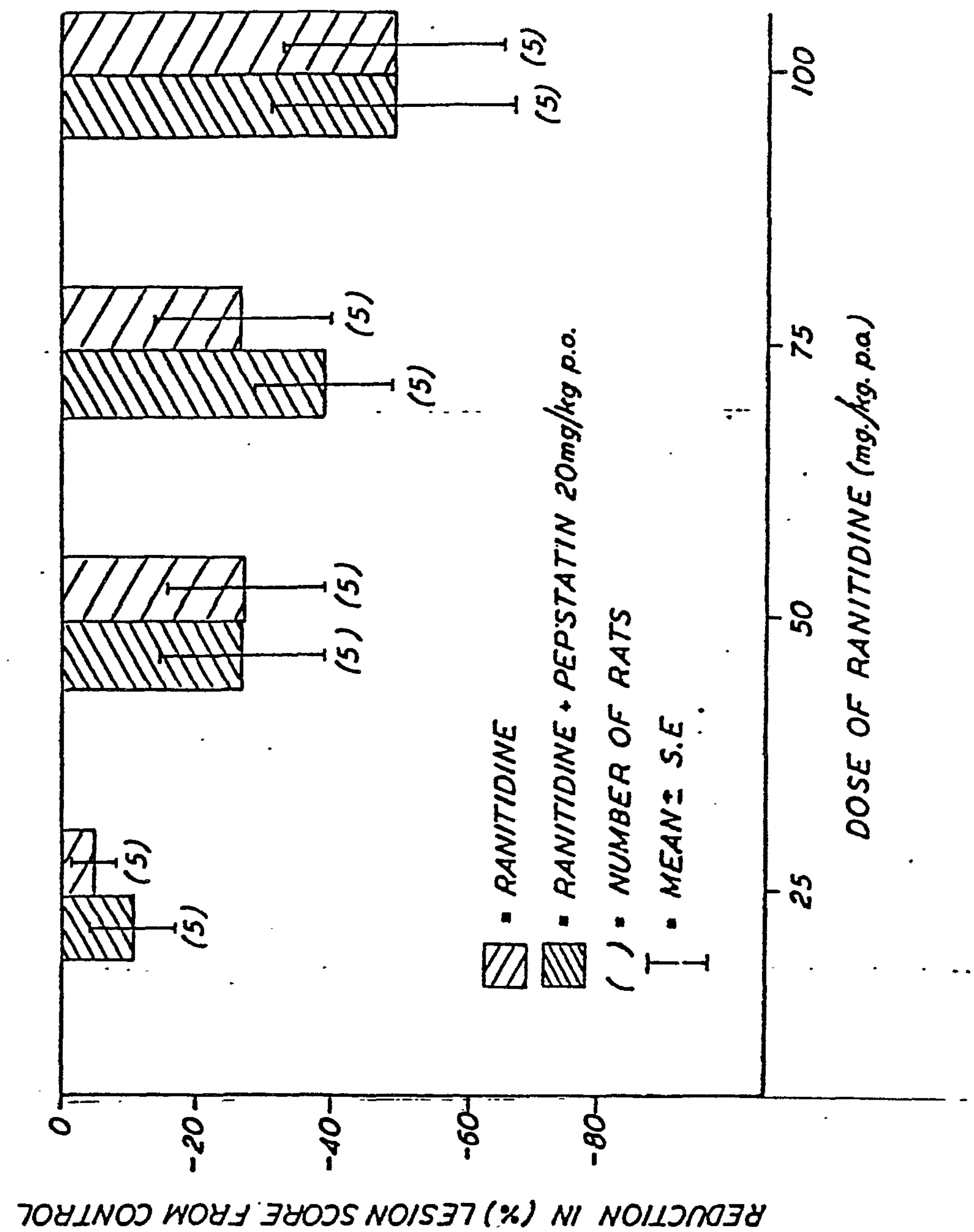

FIG. 4

DOSE RELATED EFFECTS OF RANITIDINE AND COMBINATIONS OF RANITIDINE AND PEPSTATIN (20mg./kg.) ON THE REDUCTION OF HCl INDUCED GASTRIC EROSIONS IN THE RAT.